# EUROPEAN PATENT APPLICATION

(11) **EP 2 311 867 A1**
(43) Date of publication of application: **20.04.2011**
(21) Application number: 10183260.8
(22) Date of filing: 29.10.2003
(51) Int. Cl.: C07K 14/52, A61K 38/19, C07K 14/725, C12N 15/10

(54) **Trimeric binding proteins for trimeric cytokines**

(30) Priority: 29.10.2002 DK 200201634; 29.10.2002 US 421807 P
(62) Divisional of application: 03769251.4
(71) Applicant: Anaphore, Inc., La Jolla, CA 92037 (US)
(72) Inventor: Holtet, Thor Las, 8410 Rønde (DK); Andersen, Mikkel Holmen, 8472 Sporup (DK); Ottow, Helle Krogh, 8320 Mårslet (DK)
(74) Representative: Hansen, Carsten Borgund

(57) **Abstract**

The present invention pertains to the provision of trimeric binding units which bind to trimeric cytokines. In particular there is provided a trimeric polypeptide comprising a trimerising domain and three monomers with binding members capable of binding a trimeric cytokine. Preferably, the trimeric binding units bind in a manner such that upon binding, all receptor binding sites of the trimeric cytokine are substantially blocked, and hence the potential biological activity of the trimeric cytokine is suppressed. In one aspect the invention relates to trimeric binders capable of binding to trimeric cytokines of the Tumor necrosis factor ligand superfamily, such as TNF, TRAIL, RANKL, TWEAK, APRIL and BAFF.

SEQUENCE LISTING

<110> Anaphore, Inc

<120> Trimeric binding proteins for Trimeric Cytokines

<130> 16156EP01

<160> 109

<170> PatentIn version 3.3

<210> 1
<211> 33
<212> DNA
<213> Homo sapiens

<400> 1
ctgccatgcg gccgccttca gggagaccgt ctg 33

<210> 2
<211> 58
<212> DNA
<213> Homo sapiens

<400> 2
gtgaccaggc ccagccggcc atgtaagtag ctaaggtacc gagccaccaa cccagaag 58

<210> 3
<211> 197
<212> DNA
<213> Homo sapiens

<400> 3
ggcccagccg gccatgtaag tagctaaggt accgagccac caacccagaa gcccaagaag 60

attgtaaatg ccaagaaaga tgttgtgaac acaaagatgt ttgaggagct caagagccgt 120

ctggacaccc tggcccagga ggtggccctg ctgaaggagc agcaggccct gcagacggtc 180

tccctgaagg cggccgc 197

<210> 4
<211> 62
<212> DNA
<213> Homo sapiens

<400> 4
gtgaccaggc ccagccggcc atgtaagtag ctaaggtacc attgtaaatg ccaagaaaga 60

tg 62

<210> 5
<211> 170
<212> DNA
<213> Homo sapiens

<400> 5
ggcccagccg gccatgtaag tagctaaggt accattgtaa atgccaagaa agatgttgtg 60

aacacaaaga tgtttgagga gctcaagagc cgtctggaca ccctggccca ggaggtggcc 120

ctgctgaagg agcagcaggc cctgcagacg gtctccctga aggcggccgc 170

<210> 6
<211> 32
<212> DNA
<213> Homo sapiens

<400> 6
ctgccataag cttacttcag ggagaccgtc tg 32


<210> 7
<211> 51
<212> DNA
<213> Homo sapiens

<400> 7
gtgaccagga tccatgtaag tagctaaggt accgagccac caacccagaa g 51


<210> 8
<211> 191
<212> DNA
<213> Homo sapiens

<400> 8
ggatccatgt aagtagctaa ctgcaggtga gccaccaacc cagaagccca agaagattgt 60

aaatgccaag aaagatgttg tgaacacaaa gatgtttgag gagctcaaga gccgtctgga 120

caccctggcc caggaggtgg ccctgctgaa ggagcagcag gccctgcaga cggtctccct 180

gaagtaagct t 191


<210> 9
<211> 55
<212> DNA
<213> Homo sapiens

<400> 9
gtgaccagga tccatgtaag tagctaaggt accattgtaa atgccaagaa agatg 55


<210> 10
<211> 162
<212> DNA
<213> Homo sapiens

<400> 10
ggatccatgt aagtagctaa ggtaccattg taaatgccaa gaaagatgtt gtgaacacaa 60

agatgtttga ggagctcaag agccgtctgg acaccctggc ccaggaggtg gccctgctga 120

aggagcagca ggccctgcag acggtctccc tgaagtaagc tt 162


<210> 11
<211> 44
<212> DNA
<213> Homo sapiens

<400> 11
gtgaccaggc ccagccggcc atggccggga gcacatgccg gctc 44


<210> 12
<211> 34
<212> DNA

<213>  Homo sapiens

<400>  12
cgtgctgggt accggtgcag atgcggttct gttc                                    34


<210>  13
<211>  274
<212>  DNA
<213>  Homo sapiens

<400>  13
ggcccagccg gccatggccg ggagcacatg ccggctcaga gaatactatg accagacagc        60

tcagatgtgc tgcagcaagt gctcgccggg ccaacatgca aaagtcttct gtaccaagac       120

ctcggacacc gtgtgtgact cctgtgagga cagcacatac acccagctct ggaactgggt       180

tcccgagtgc ttgagctgtg gctcccgctg tagctctgac caggtggaaa ctcaagcctg       240

cactcgggaa cagaaccgca tctgcaccgg tacc                                   274


<210>  14
<211>  39
<212>  DNA
<213>  Homo sapiens

<400>  14
cgtgctgggt accgggccta gcggtgcaga tgcggttct                               39


<210>  15
<211>  283
<212>  DNA
<213>  Homo sapiens

<400>  15
ggcccagccg gccatggccg ggagcacatg ccggctcaga gaatactatg accagacagc        60

tcagatgtgc tgcagcaagt gctcgccggg ccaacatgca aaagtcttct gtaccaagac       120

ctcggacacc gtgtgtgact cctgtgagga cagcacatac acccagctct ggaactgggt       180

tcccgagtgc ttgagctgtg gctcccgctg tagctctgac caggtggaaa ctcaagcctg       240

cactcgggaa cagaaccgca tctgcaccgc taggcccggt acc                         283


<210>  16
<211>  31
<212>  DNA
<213>  Homo sapiens

<400>  16
cgtgctgggt acccgcgcag taccagccgg g                                       31


<210>  17
<211>  298
<212>  DNA
<213>  Homo sapiens

<400>  17
ggcccagccg gccatggccg ggagcacatg ccggctcaga gaatactatg accagacagc        60

tcagatgtgc tgcagcaagt gctcgccggg ccaacatgca aaagtcttct gtaccaagac       120

ctcggacacc gtgtgtgact cctgtgagga cagcacatac acccagctct ggaactgggt 180

tcccgagtgc ttgagctgtg gctcccgctg tagctctgac caggtggaaa ctcaagcctg 240

cactcgggaa cagaaccgca tctgcacctg caggcccggc tggtactgcg cgggtacc 298

<210> 18
<211> 29
<212> DNA
<213> Homo sapiens

<400> 18
cgtgctgggt accctcctgc ttgctcagc 29

<210> 19
<211> 316
<212> DNA
<213> Homo sapiens

<400> 19
ggcccagccg gccatggccg ggagcacatg ccggctcaga gaatactatg accagacagc 60

tcagatgtgc tgcagcaagt gctcgccggg ccaacatgca aaagtcttct gtaccaagac 120

ctcggacacc gtgtgtgact cctgtgagga cagcacatac acccagctct ggaactgggt 180

tcccgagtgc ttgagctgtg gctcccgctg tagctctgac caggtggaaa ctcaagcctg 240

cactcgggaa cagaaccgca tctgcacctg caggcccggc tggtactgcg cgctgagcaa 300

gcaggagggg ggtacc 316

<210> 20
<211> 44
<212> DNA
<213> Homo sapiens

<400> 20
cgtgctgggt acctgcagcc agccgagccc cctcctgctt gctc 44

<210> 21
<211> 331
<212> DNA
<213> Homo sapiens

<400> 21
ggcccagccg gccatggccg ggagcacatg ccggctcaga gaatactatg accagacagc 60

tcagatgtgc tgcagcaagt gctcgccggg ccaacatgca aaagtcttct gtaccaagac 120

ctcggacacc gtgtgtgact cctgtgagga cagcacatac acccagctct ggaactgggt 180

tcccgagtgc ttgagctgtg gctcccgctg tagctctgac caggtggaaa ctcaagcctg 240

cactcgggaa cagaaccgca tctgcacctg caggcccggc tggtactgcg cgctgagcaa 300

gcaggagggg gctcggctgg ctgcaggtac c 331

<210> 22
<211> 31
<212> DNA
<213> Homo sapiens

<400> 22
cgtgctgggt accggacgtg gacgtgcaga c 31

<210> 23
<211> 529
<212> DNA
<213> Homo sapiens

<400> 23
ggcccagccg gccatggccg ggagcacatg ccggctcaga gaatactatg accagacagc 60

tcagatgtgc tgcagcaagt gctcgccggg ccaacatgca aaagtcttct gtaccaagac 120

ctcggacacc gtgtgtgact cctgtgagga cagcacatac acccagctct ggaactgggt 180

tcccgagtgc ttgagctgtg gctcccgctg tagctctgac caggtggaaa ctcaagcctg 240

cactcgggaa cagaaccgca tctgcacctg caggcccggc tggtactgcg cgctgagcaa 300

gcaggagggg tgccggctgt gcgcgccgct gcgcaagtgc cgcccgggct tcggcgtggc 360

cagaccagga actgaaacat cagacgtggt gtgcaagccc tgtgccccgg ggacgttctc 420

caacacgact tcatccacgg atatttgcag gccccaccag atctgtaacg tggtggccat 480

ccctgggaat gcaagcaggg atgcagtctg cacgtccacg tccggtacc 529

<210> 24
<211> 44
<212> DNA
<213> Homo sapiens

<400> 24
gtgaccaggc ccagccggcc atggccgact cctgtgagga cagc 44

<210> 25
<211> 157
<212> DNA
<213> Homo sapiens

<400> 25
ggcccagccg gccatggccg actcctgtga ggacagcaca tacacccagc tctggaactg 60

ggttcccgag tgcttgagct gtggctcccg ctgtagctct gaccaggtgg aaactcaagc 120

ctgcactcgg gaacagaacc gcatctgcac cggtacc 157

<210> 26
<211> 166
<212> DNA
<213> Homo sapiens

<400> 26
ggcccagccg gccatggccg actcctgtga ggacagcaca tacacccagc tctggaactg 60

ggttcccgag tgcttgagct gtggctcccg ctgtagctct gaccaggtgg aaactcaagc 120

ctgcactcgg gaacagaacc gcatctgcac cgctaggccc ggtacc 166

<210> 27
<211> 182
<212> DNA
<213> Homo sapiens

<400> 27
ggcccagccg gccatggcct gactcctgtg aggacagcac atacacccag ctctggaact 60

gggttcccga gtgcttgagc tgtggctccc gctgtagctc tgaccaggtg gaaactcaag 120

cctgcactcg ggaacagaac cgcatctgca cctgcaggcc cggctggtac tgcgcgggta 180

cc 182

<210> 28
<211> 199
<212> DNA
<213> Homo sapiens

<400> 28
   ggcccagccg gccatggccg actcctgtga ggacagcaca tacacccagc tctggaactg 60
   ggttcccgag tgcttgagct gtggctcccg ctgtagctct gaccaggtgg aaactcaagc 120
   ctgcactcgg gaacagaacc gcatctgcac ctgcaggccc ggctggtact gcgcgctgag 180
   caagcaggag gggggtacc 199

<210> 29
<211> 214
<212> DNA
<213> Homo sapiens

<400> 29
ggcccagccg gccatggccg actcctgtga ggacagcaca tacacccagc tctggaactg 60

ggttcccgag tgcttgagct gtggctcccg ctgtagctct gaccaggtgg aaactcaagc 120

ctgcactcgg gaacagaacc gcatctgcac ctgcaggccc ggctggtact gcgcgctgag 180

caagcaggag ggggctcggc tggctgcagg tacc 214

<210> 30
<211> 412
<212> DNA
<213> Homo sapiens

<400> 30
ggcccagccg gccatggccg actcctgtga ggacagcaca tacacccagc tctggaactg 60

ggttcccgag tgcttgagct gtggctcccg ctgtagctct gaccaggtgg aaactcaagc 120

ctgcactcgg gaacagaacc gcatctgcac ctgcaggccc ggctggtact gcgcgctgag 180

caagcaggag gggtgccggc tgtgcgcgcc gctgcgcaag tgccgcccgg gcttcggcgt 240

ggccagacca ggaactgaaa catcagacgt ggtgtgcaag ccctgtgccc cggggacgtt 300

ctccaacacg acttcatcca cggatatttg caggccccac cagatctgta acgtggtggc 360

catccctggg aatgcaagca gggatgcagt ctgcacgtcc acgtccggta cc 412

<210> 31
<211> 41
<212> DNA
<213> Homo sapiens

<400> 31

gccagggatc catcgagggt agggggagca catgccggct c 41

<210> 32
<211> 278
<212> DNA
<213> Homo sapiens

<400> 32
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca ccggtacc 278

<210> 33
<211> 287
<212> DNA
<213> Homo sapiens

<400> 33
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca ccgctaggcc cggtacc 287

<210> 34
<211> 302
<212> DNA
<213> Homo sapiens

<400> 34
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca cctgcaggcc cggctggtac tgcgcgggta 300

cc 302

<210> 35
<211> 320
<212> DNA
<213> Homo sapiens

<400> 35
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca cctgcaggcc cggctggtac tgcgcgctga 300

gcaagcagga ggggggtacc 320

<210> 36
<211> 335
<212> DNA
<213> Homo sapiens

<400> 36
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca cctgcaggcc cggctggtac tgcgcgctga 300

gcaagcagga gggggctcgg ctggctgcag gtacc 335

<210> 37
<211> 533
<212> DNA
<213> Homo sapiens

<400> 37
gccagggatc catcgagggt agggggagca catgccggct cagagaatac tatgaccaga 60

cagctcagat gtgctgcagc aagtgctcgc cgggccaaca tgcaaaagtc ttctgtacca 120

agacctcgga caccgtgtgt gactcctgtg aggacagcac atacacccag ctctggaact 180

gggttcccga gtgcttgagc tgtggctccc gctgtagctc tgaccaggtg gaaactcaag 240

cctgcactcg ggaacagaac cgcatctgca cctgcaggcc cggctggtac tgcgcgctga 300

gcaagcagga ggggtgccgg ctgtgcgcgc cgctgcgcaa gtgccgcccg ggcttcggcg 360

tggccagacc aggaactgaa acatcagacg tggtgtgcaa gccctgtgcc ccggggacgt 420

tctccaacac gacttcatcc acggatattt gcaggcccca ccagatctgt aacgtggtgg 480

ccatccctgg gaatgcaagc agggatgcag tctgcacgtc cacgtccggt acc 533

<210> 38
<211> 44
<212> DNA
<213> Homo sapiens

<400> 38
gccagggatc catcgagggt aggggcgact cctgtgagga cagc 44

<210> 39
<211> 159
<212> DNA
<213> Homo sapiens

<400> 39
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc accggtacc 159

<210> 40
<211> 168
<212> DNA
<213> Homo sapiens

<400> 40
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc accgctaggc ccggtacc 168

<210> 41
<211> 183
<212> DNA
<213> Homo sapiens

<400> 41
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc acctgcaggc ccggctggta ctgcgcgggt 180

acc 183

<210> 42
<211> 201
<212> DNA
<213> Homo sapiens

<400> 42
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc acctgcaggc ccggctggta ctgcgcgctg 180

agcaagcagg aggggggtac c 201

<210> 43
<211> 216
<212> DNA
<213> Homo sapiens

<400> 43
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc acctgcaggc ccggctggta ctgcgcgctg 180

agcaagcagg aggggctcgg ctggctgcag ggtacc 216

<210> 44
<211> 414
<212> DNA
<213> Homo sapiens

<400> 44
ggatccatcg agggtagggg cgactcctgt gaggacagca catacaccca gctctggaac 60

tgggttcccg agtgcttgag ctgtggctcc cgctgtagct ctgaccaggt ggaaactcaa 120

gcctgcactc gggaacagaa ccgcatctgc acctgcaggc ccggctggta ctgcgcgctg 180

agcaagcagg aggggtgccg gctgtgcgcg ccgctgcgca agtgccgccc gggcttcggc 240

gtggccagac caggaactga aacatcagac gtggtgtgca agccctgtgc cccggggacg 300

ttctccaaca cgacttcatc cacggatatt tgcaggcccc accagatctg taacgtggtg 360

gccatccctg ggaatgcaag cagggatgca gtctgcacgt ccacgtccgg tacc 414


<210> 45
<211> 41
<212> DNA
<213> Homo sapiens

<400> 45
gccagagatc tatcgagggt agggaggtgc agctggtgga g 41


<210> 46
<211> 59
<212> DNA
<213> Homo sapiens

<400> 46
ctggtcggta ccggatccgc cgccaccact cgagacggtg accagagtac cttggcccc 59


<210> 47
<211> 62
<212> DNA
<213> Homo sapiens

<400> 47
gcaggcggat ccgggggagg aggtagtggc ggtggtggat cagacatcca gatgacccag 60

tc 62


<210> 48
<211> 33
<212> DNA
<213> Homo sapiens

<400> 48
ccgagcggta cctttgattt ccaccttggt ccc 33


<210> 49
<211> 754
<212> DNA
<213> Homo sapiens

<400> 49
agatctatcg agggtaggga ggtgcagctg gtggagtctg ggggaggctt ggtacagccc 60

ggcaggtccc tgagactctc ctgtgcggcc tctggattca cctttgatga ttatgccatg 120

cactgggtcc ggcaagctcc agggaagggc ctggaatggg tctcagctat cacttggaat 180

agtggtcaca tagactatgc ggactctgtg gagggccgat tcaccatctc cagagacaac 240

gccaagaact ccctgtatct gcaaatgaac agtctgagag ctgaggatac ggccgtatat 300

tactgtgcga aagtctcgta ccttagcacc gcgtcctccc ttgactattg gggccaaggt 360

accctggtca ccgtctcgag tggtggcggc gggatccggg ggaggaggta gtggcggtgg 420

tggatcagac atccagatga cccagtctcc atcctccctg tctgcatctg taggggacag 480

agtcaccatc acttgtcggg caagtcaggg catcagaaat tacttagcct ggtatcagca 540

aaaaccaggg aaagccccta agctcctgat ctatgctgca tccactttgc aatcaggggt 600

cccatctcgg ttcagtggca gtggatctgg gacagatttc actctcacca tcagcagcct 660

acagcctgaa gatgttgcaa cttattactg tcaaaggtat aaccgtgcac cgtatacttt 720

tggccagggg accaaggtgg aaatcaaagg tacc 754

<210> 50
<211> 48
<212> DNA
<213> Homo sapiens

<400> 50
ccgagcggta ccagatccac cgcccccttt gatttccacc ttggtccc 48

<210> 51
<211> 769
<212> DNA
<213> Homo sapiens

<400> 51
agatctatcg agggtaggga ggtgcagctg gtggagtctg gggaggcttg ggtacagccc 60

ggcaggtccc tgagactctc ctgtgcggcc tctggattca cctttgatga ttatgccatg 120

cactgggtcc ggcaagctcc agggaagggc ctggaatggg tctcagctat cacttggaat 180

agtggtcaca tagactatgc ggactctgtg gagggccgat tcaccatctc cagagacaac 240

gccaagaact ccctgtatct gcaaatgaac agtctgagag ctgaggatac ggccgtatat 300

tactgtgcga aagtctcgta ccttagcacc gcgtcctccc ttgactattg gggccaaggt 360

accctggtca ccgtctcgag tggtggcggc gggatccggg ggaggaggta gtggcggtgg 420

tggatcagac atccagatga cccagtctcc atcctccctg tctgcatctg taggggacag 480

agtcaccatc acttgtcggg caagtcaggg catcagaaat tacttagcct ggtatcagca 540

aaaaccaggg aaagccccta agctcctgat ctatgctgca tccactttgc aatcaggggt 600

cccatctcgg ttcagtggca gtggatctgg gacagatttc actctcacca tcagcagcct 660

acagcctgaa gatgttgcaa cttattactg tcaaaggtat aaccgtgcac cgtatacttt 720

tggccagggg accaaggtgg aaatcaaagg gggcggtgga tctggtacc 769

<210> 52
<211> 29
<212> DNA
<213> Homo sapiens

<400> 52
gccagtgatc agaggtgcag ctggtggag 29

<210> 53
<211> 33
<212> DNA
<213> Homo sapiens

<400> 53
cctcgaagct tatttgattt ccaccttggt ccc 33


<210> 54
<211> 743
<212> DNA
<213> Homo sapiens

<400> 54
tgatcagagg tgcagctggt ggagtctggg ggaggcttgg tacagcccgg caggtccctg 60

agactctcct gtgcggcctc tggattcacc tttgatgatt atgccatgca ctgggtccgg 120

caagctccag ggaagggcct ggaatgggtc tcagctatca cttggaatag tggtcacata 180

gactatgcgg actctgtgga gggccgattc accatctcca gagacaacgc caagaactcc 240

ctgtatctgc aaatgaacag tctgagagct gaggatacgg ccgtatatta ctgtgcgaaa 300

gtctcgtacc ttagcaccgc gtcctccctt gactattggg gccaaggtac cctggtcacc 360

gtctcgagtg gtggcggcgg atccgggggg aggaggtagt ggcggtggtg gatcagacat 420

ccagatgacc cagtctccat cctccctgtc tgcatctgta ggggacagag tcaccatcac 480

ttgtcgggca agtcagggca tcagaaatta cttagcctgg tatcagcaaa aaccagggaa 540

agcccctaag ctcctgatct atgctgcatc cactttgcaa tcaggggtcc catctcggtt 600

cagtggcagt ggatctggga cagatttcac tctcaccatc agcagcctac agcctgaaga 660

tgttgcaact tattactgtc aaaggtataa ccgtgcaccg tatacttttg gccaggggac 720

caaggtggaa atcaaataag ctt 743


<210> 55
<211> 44
<212> DNA
<213> Homo sapiens

<400> 55
gccagtgatc aggaggtggc gggtctgagg tgcagctggt ggag 44


<210> 56
<211> 758
<212> DNA
<213> Homo sapiens

<400> 56
tgatcaggag gtggcgggtc tgaggtgcag ctggtggagt ctgggggagg cttggtacag 60

cccggcaggt ccctgagact ctcctgtgcg gcctctggat tcacctttga tgattatgcc 120

atgcactggg tccggcaagc tccagggaag ggcctggaat gggtctcagc tatcacttgg 180

aatagtggtc acatagacta tgcggactct gtggagggcc gattcaccat ctccagagac 240

aacgccaaga actccctgta tctgcaaatg aacagtctga gagctgagga tacggccgta 300

tattactgtg cgaaagtctc gtaccttagc accgcgtcct cccttgacta ttggggccaa 360

ggtaccctgg tcaccgtctc gagtggtggc ggcgggatcc gggggaggag gtagtggcgg 420

tggtggatca gacatccaga tgacccagtc tccatcctcc ctgtctgcat ctgtagggga 480

cagagtcacc atcacttgtc gggcaagtca gggcatcaga aattacttag cctggtatca 540

gcaaaaacca gggaaagccc ctaagctcct gatctatgct gcatccactt tgcaatcagg 600

ggtcccatct cggttcagtg gcagtggatc tgggacagat ttcactctca ccatcagcag 660

cctacagcct gaagatgttg caacttatta ctgtcaaagg tataaccgtg caccgtatac 720

ttttggccag gggaccaagg tggaaatcaa ataagctt 758

<210> 57
<211> 38
<212> DNA
<213> Homo sapiens

<400> 57
gccagagatc tatcgagggt aggatgagtg gcctgggc 38

<210> 58
<211> 18
<212> DNA
<213> Homo sapiens

<400> 58
catgcaggta cccagcag 18

<210> 59
<211> 18
<212> DNA
<213> Homo sapiens

<400> 59
ctgctgggta cctgcatg 18

<210> 60
<211> 32
<212> DNA
<213> Homo sapiens

<400> 60
cggcacggta ccgctcctga gcttgttctc ac 32

<210> 61
<211> 354
<212> DNA
<213> Homo sapiens

<400> 61
agatctatcg agggtaggat gagtggcctg ggccggagca ggcgaggtgg ccggagccgt 60

gtggaccagg aggagcgctt tccacagggc ctgtggacgg gggtggctat gagatcctgc 120

cccgaagagc agtactggga tcctctgctg ggtacctgca tgtcctgcaa aaccatttgc 180

aaccatcaga gccagcgcac ctgtgcagcc ttctgcaggt cactcagctg ccgcaaggag 240

caaggcaagt tctatgacca tctcctgagg gactgcatca gctgtgcctc catctgtgga 300

cagcacccta agcaatgtgc atacttctgt gagaacaagc tcaggagcgg tacc 354

<210> 62
<211> 31
<212> DNA
<213> Homo sapiens

<400> 62
cggcacggta ccaaggttca ctgggctcct g 31

<210> 63
<211> 366
<212> DNA
<213> Homo sapiens

<400> 63
agatctatcg agggtaggat gagtggcctg ggccggagca ggcgaggtgg ccggagccgt 60

gtggaccagg aggagcgctt tccacagggc ctgtggacgg gggtggctat gagatcctgc 120

cccgaagagc agtactggga tcctctgctg ggtacctgca tgtcctgcaa aaccatttgc 180

aaccatcaga gccagcgcac ctgtgcagcc ttctgcaggt cactcagctg ccgcaaggag 240

caaggcaagt tctatgacca tctcctgagg gactgcatca gctgtgcctc catctgtgga 300

cagcacccta agcaatgtgc atacttctgt gagaacaagc tcaggagccc agtgaacctt 360

ggtacc 366

<210> 64
<211> 31
<212> DNA
<213> Homo sapiens

<400> 64
cggcacggta cctccactcc gctgtctcct g 31

<210> 65
<211> 396
<212> DNA
<213> Homo sapiens

<400> 65
agatctatcg agggtaggat gagtggcctg ggccggagca ggcgaggtgg ccggagccgt 60

gtggaccagg aggagcgctt tccacagggc ctgtggacgg gggtggctat gagatcctgc 120

cccgaagagc agtactggga tcctctgctg ggtacctgca tgtcctgcaa aaccatttgc 180

aaccatcaga gccagcgcac ctgtgcagcc ttctgcaggt cactcagctg ccgcaaggag 240

caaggcaagt tctatgacca tctcctgagg gactgcatca gctgtgcctc catctgtgga 300

cagcacccta agcaatgtgc atacttctgt gagaacaagc tcaggagccc agtgaacctt 360

ccaccagagc tcaggagaca gcggagtgga ggtacc 396

<210> 66
<211> 36
<212> DNA

<213>  Homo sapiens

<400>  66
cggcacggta ccagggctca acagacttaa caaaag                                36


<210>  67
<211>  426
<212>  DNA
<213>  Homo sapiens

<400>  67
agatctatcg agggtaggat gagtggcctg ggccggagca ggcgaggtgg ccggagccgt      60

gtggaccagg aggagcgctt tccacagggc ctgtggacgg gggtggctat gagatcctgc     120

cccgaagagc agtactggga tcctctgctg ggtacctgca tgtcctgcaa aaccatttgc     180

aaccatcaga gccagcgcac ctgtgcagcc ttctgcaggt cactcagctg ccgcaaggag     240

caaggcaagt tctatgacca tctcctgagg gactgcatca gctgtgcctc catctgtgga     300

cagcacccta agcaatgtgc atacttctgt gagaacaagc tcaggagccc agtgaacctt     360

ccaccagagc tcaggagaca gcggagtgga gaagttgaaa acaattcaga caactcggga     420

ggtacc                                                                426


<210>  68
<211>  31
<212>  DNA
<213>  Homo sapiens

<400>  68
cggcacggta ccgctgtaga ccagggccac c                                     31


<210>  69
<211>  519
<212>  DNA
<213>  Homo sapiens

<400>  69
agatctatcg agggtaggat gagtggcctg ggccggagca ggcgaggtgg ccggagccgt      60

gtggaccagg aggagcgctt tccacagggc ctgtggacgg gggtggctat gagatcctgc     120

cccgaagagc agtactggga tcctctgctg ggtacctgca tgtcctgcaa aaccatttgc     180

aaccatcaga gccagcgcac ctgtgcagcc ttctgcaggt cactcagctg ccgcaaggag     240

caaggcaagt tctatgacca tctcctgagg gactgcatca gctgtgcctc catctgtgga     300

cagcacccta agcaatgtgc atacttctgt gagaacaagc tcaggagccc agtgaacctt     360

ccaccagagc tcaggagaca gcggagtgga gaagttgaaa acaattcaga caactcggga     420

aggtaccaag gattggagca cagaggctca gaagcaagtc cagctctccc ggggctgaag     480

ctgagtgcag atcaggtggc cctggtctac agcggtacc                            519


<210>  70
<211>  41
<212>  DNA
<213>  Homo sapiens

<400>  70
ggccagggat  ccatcgaggg  taggggggag  caagcgccag  g                    41

<210>  71
<211>  31
<212>  DNA
<213>  Homo sapiens

<400>  71
cggtgcggta  ccgggccaaa  gcagccggaa  g                               31

<210>  72
<211>  186
<212>  DNA
<213>  Homo sapiens

<400>  72
ggatccatcg  agggtagggg  ggagcaagcg  ccaggcaccg  ccccctgctc  ccgcggcagc      60

tcctggagcg  cggacctgga  caagtgcatg  gactgcgcgt  cttgcagggc  gcgaccgcac     120

agcgacttct  gcctgggctg  cgctgcagca  cctcctgccc  ccttccggct  gctttggccc     180

ggtacc                                                              186

<210>  73
<211>  41
<212>  DNA
<213>  Homo sapiens

<400>  73
ggccagggat  ccatcgaggg  taggatgagg  cgagggcccc  g                    41

<210>  74
<211>  28
<212>  DNA
<213>  Homo sapiens

<400>  74
cggtgcggta  ccgagcagcc  cgggcagg                                    28

<210>  75
<211>  258
<212>  DNA
<213>  Homo sapiens

<400>  75
ggatccatcg  agggtaggat  gaggcgaggg  ccccggagcc  tgcggggcag  ggacgcgcca      60

gcccccacgc  cctgcgtccc  ggccgagtgc  ttcgacctgc  tggtccgcca  ctgcgtggcc     120

tgcgggctcc  tgcgcacgcc  gcggccgaaa  ccggccgggg  ccagcagccc  tgcgcccagg     180

acggcgctgc  agccgcagga  gtcggtgggc  gcgggggccg  gcgaggcggc  gctgcccctg     240

cccgggctgc  tcggtacc                                                258

<210>  76
<211>  235
<212>  PRT
<213>  Homo sapiens

<400> 76

Leu Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala
        115                 120                 125

Arg Pro Gly Thr Glu Thr Ser Asp Val Val Cys Lys Pro Cys Ala Pro
    130                 135                 140

Gly Thr Phe Ser Asn Thr Thr Ser Ser Thr Asp Ile Cys Arg Pro His
145                 150                 155                 160

Gln Ile Cys Asn Val Val Ala Ile Pro Gly Asn Ala Ser Met Asp Ala
                165                 170                 175

Val Cys Thr Ser Thr Ser Pro Thr Arg Ser Met Ala Pro Gly Ala Val
            180                 185                 190

His Leu Pro Gln Pro Val Ser Thr Arg Ser Gln His Thr Gln Pro Thr
        195                 200                 205

Pro Glu Pro Ser Thr Ala Pro Ser Thr Ser Phe Leu Leu Pro Met Gly
    210                 215                 220

Pro Ser Pro Pro Ala Glu Gly Ser Thr Gly Asp
225                 230                 235

<210>  77
<211>  185
<212>  PRT
<213>  Homo sapiens

<400>  77

Leu Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala
        115                 120                 125

Arg Pro Gly Thr Glu Thr Ser Asp Val Val Cys Lys Pro Cys Ala Pro
    130                 135                 140

Gly Thr Phe Ser Asn Thr Thr Ser Ser Thr Asp Ile Cys Arg Pro His
145                 150                 155                 160

Gln Ile Cys Asn Val Val Ala Ile Pro Gly Asn Ala Ser Met Asp Ala
                165                 170                 175

Val Cys Thr Ser Thr Ser Pro Thr Arg
            180                 185

<210>  78
<211>  163
<212>  PRT
<213>  Homo sapiens

<400>  78

Leu Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala
        115                 120                 125

Arg Pro Gly Thr Glu Thr Ser Asp Val Val Cys Lys Pro Cys Ala Pro
    130                 135                 140

Gly Thr Phe Ser Asn Thr Thr Ser Ser Thr Asp Ile Cys Arg Pro His
145                 150                 155                 160

Gln Ile Cys

<210> 79
<211> 142
<212> PRT
<213> Homo sapiens

<400> 79

Leu Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala

115                 120                 125

Arg Pro Gly Thr Glu Thr Ser Asp Val Val Cys Lys Pro Cys
    130                 135                 140

<210>  80
<211>  157
<212>  PRT
<213>  Homo sapiens

<400>  80

<210>  81
<211>  51
<212>  PRT
<213>  Homo sapiens

<400>  81

Glu Pro Pro Thr Gln Lys Pro Lys Lys Leu Val Asn Ala Lys Lys Asp
1               5                   10                  15

Val Val Asn Thr Lys Met Phe Glu Glu Leu Lys Ser Arg Leu Asp Thr
            20                  25                  30

Leu Ala Gln Glu Val Ala Leu Leu Lys Glu Gln Gln Ala Leu Gln Thr
        35                  40                  45

Val Cys Leu
    50

<210> 82
<211> 39
<212> DNA
<213> Artificial

<220>
<223> oligonucleotide primer

<400> 82
gcgcacggat ccatggccca ggtggcattt acaccctac                              39

<210> 83
<211> 33
<212> DNA
<213> Artificial

<220>
<223> oligonucleotide primer

<400> 83
caccacggta ccgatctggt ggggcctgca aat                                    33

<210> 84
<211> 738
<212> DNA
<213> Artificial

<220>
<223> AD1D4-I162-tripB

<400> 84
atgggatcca tggcccaggt ggcatttaca ccctacgccc cggagcccgg gagcacatgc      60

cggctcagag aatactatga ccagacagct cagatgtgct gcagcaaatg ctcgccgggc     120

caacatgcaa aagtcttctg taccaagacc tcggacaccg tgtgtgactc ctgtgaggac     180

agcacataca cccagctctg gaactgggtt cccgagtgct tgagctgtgg ctcccgctgt     240

agctctgacc aggtggaaac tcaagcctgc actcgggaac agaaccgcat ctgcacctgc     300

aggcccggct ggtactgcgc gctgagcaag caggaggggt gccggctgtg cgcgccgctg     360

cgcaagtgcc gcccgggctt cggcgtggcc agaccaggaa ctgaaacatc agacgtggtg     420

tgcaagccct gtgccccggg gacgttctcc aacacgactt catccacgga tatttgcagg     480

ccccaccaga tcggtaccga gccaccaacc cagaagccca agaagattgt aaatgccaag     540

aaagatgttg tgaacacaaa gatgtttgag gagctcaaga gccgtctgga caccctggcc     600

caggaggtgg ccctgctgaa ggagcagcag gccctgcaga cggtctccct gaagggtcta     660

gaacaaaaac tcatctcaga agaggatctg aatagcgccg tcgaccatca tcatcatcat     720

cattgaaagc tgaattcc                                                   738

<210> 85
<211> 51
<212> DNA
<213> Artificial

<220>
<223> oligonucleotide primer

<400> 85
caccacggta ccggaggaac cggaggacgt ggacgtgcag actgcatcca t 51


<210> 86
<211> 810
<212> DNA
<213> Artificial

<220>
<223> AD1D4-GSS-tripB

<400> 86
atgggatcca tggcccaggt ggcatttaca ccctacgccc cggagcccgg gagcacatgc 60

cggctcagag aatactatga ccagacagct cagatgtgct gcagcaaatg ctcgccgggc 120

caacatgcaa aagtcttctg taccaagacc tcggacaccg tgtgtgactc ctgtgaggac 180

agcacataca cccagctctg gaactgggtt cccgagtgct tgagctgtgg ctcccgctgt 240

agctctgacc aggtggaaac tcaagcctgc actcgggaac agaaccgcat ctgcacctgc 300

aggcccggct ggtactgcgc gctgagcaag caggaggggt gccggctgtg cgcgccgctg 360

cgcaagtgcc gcccgggctt cggcgtggcc agaccaggaa ctgaaacatc agacgtggtg 420

tgcaagccct gtgccccggg gacgttctcc aacacgactt catccacgga tatttgcagg 480

ccccaccaga tctgtaacgt ggtggccatc cctgggaatg caagcatgga tgcagtctgc 540

acgtccacgt cctccggttc ctccggtacc gagccaccaa cccagaagcc caagaagatt 600

gtaaatgcca agaaagatgt tgtgaacaca aagatgtttg aggagctcaa gagccgtctg 660

gacaccctgg cccaggaggt ggccctgctg aaggagcagc aggccctgca gacggtctcc 720

ctgaagggtc tagaacaaaa actcatctca gaagaggatc tgaatagcgc cgtcgaccat 780

catcatcatc atcattgaaa gctgaattcc 810


<210> 87
<211> 39
<212> DNA
<213> Artificial

<220>
<223> oligonucleotide primer

<400> 87
agatttggta ccgtcgccag tgctcccttc agctggggg 39


<210> 88
<211> 957
<212> DNA
<213> Artificial

<220>
<223>   AD1D4-D235-tripB

<400>   88
atgggatcca tggcccaggt ggcatttaca ccctacgccc cggagcccgg gagcacatgc      60

cggctcagag aatactatga ccagacagct cagatgtgct gcagcaaatg ctcgccgggc     120

caacatgcaa aagtcttctg taccaagacc tcggacaccg tgtgtgactc ctgtgaggac     180

agcacataca cccagctctg gaactgggtt cccgagtgct tgagctgtgg ctcccgctgt     240

agctctgacc aggtggaaac tcaagcctgc actcgggaac agaaccgcat ctgcacctgc     300

aggcccggct ggtactgcgc gctgagcaag caggaggggt gccggctgtg cgcgccgctg     360

cgcaagtgcc gcccgggctt cggcgtggcc agaccaggaa ctgaaacatc agacgtggtg     420

tgcaagccct gtgccccggg gacgttctcc aacacgactt catccacgga tatttgcagg     480

ccccaccaga tctgtaacgt ggtggccatc cctgggaatg caagcatgga tgcagtctgc     540

acgtccacgt cccccacccg gagtatggcc ccaggggcag tacacttacc ccagccagtg     600

tccacacgat cccaacacac gcagccaact ccagaaccca gcactgctcc aagcacctcc     660

ttcctgctcc caatgggccc cagcccccca gctgaaggga gcactggcga cggtaccgag     720

ccaccaaccc agaagcccaa gaagattgta aatgccaaga aagatgttgt gaacacaaag     780

atgtttgagg agctcaagag ccgtctggac accctggccc aggaggtggc cctgctgaag     840

gagcagcagg ccctgcagac ggtctccctg aagggtctag aacaaaaact catctcagaa     900

gaggatctga atagcgccgt cgaccatcat catcatcatc attgaaagct gaattcc        957

<210>   89
<211>   711
<212>   DNA
<213>   Artificial

<220>
<223>   AD1D4-I162-I10-TripB

<400>   89
atgggatcca tggcccaggt ggcatttaca ccctacgccc cggagcccgg gagcacatgc      60

cggctcagag aatactatga ccagacagct cagatgtgct gcagcaaatg ctcgccgggc     120

caacatgcaa aagtcttctg taccaagacc tcggacaccg tgtgtgactc ctgtgaggac     180

agcacataca cccagctctg gaactgggtt cccgagtgct tgagctgtgg ctcccgctgt     240

agctctgacc aggtggaaac tcaagcctgc actcgggaac agaaccgcat ctgcacctgc     300

aggcccggct ggtactgcgc gctgagcaag caggaggggt gccggctgtg cgcgccgctg     360

cgcaagtgcc gcccgggctt cggcgtggcc agaccaggaa ctgaaacatc agacgtggtg     420

tgcaagccct gtgccccggg gacgttctcc aacacgactt catccacgga tatttgcagg     480

ccccaccaga tcggtaccat tgtaaatgcc aagaaagatg ttgtgaacac aaagatgttt     540

gaggagctca agagccgtct ggacaccctg gcccaggagg tggccctgct gaaggagcag     600

caggccctgc agacggtctc cctgaagggt ctagaacaaa aactcatctc agaagaggat     660

ctgaatagcg ccgtcgacca tcatcatcat catcattgaa agctgaattc c 711

<210> 90
<211> 711
<212> DNA
<213> Artificial

<220>
<223> AD1D4-GSS-I10-tripB

<400> 90
atgggatcca tggcccaggt ggcatttaca ccctacgccc cggagcccgg gagcacatgc 60

cggctcagag aatactatga ccagacagct cagatgtgct gcagcaaatg ctcgccgggc 120

caacatgcaa aagtcttctg taccaagacc tcggacaccg tgtgtgactc ctgtgaggac 180

agcacataca cccagctctg gaactgggtt cccgagtgct tgagctgtgg ctcccgctgt 240

agctctgacc aggtggaaac tcaagcctgc actcgggaac agaaccgcat ctgcacctgc 300

aggcccggct ggtactgcgc gctgagcaag caggaggggt gccggctgtg cgcgccgctg 360

cgcaagtgcc gcccgggctt cggcgtggcc agaccaggaa ctgaaacatc agacgtggtg 420

tgcaagccct gtgccccggg gacgttctcc aacacgactt catccacgga tatttgcagg 480

ccccaccaga tcggtaccat tgtaaatgcc aagaaagatg ttgtgaacac aaagatgttt 540

gaggagctca agagccgtct ggacaccctg gcccaggagg tggccctgct gaaggagcag 600

caggccctgc agacggtctc cctgaagggt ctagaacaaa aactcatctc agaagaggat 660

ctgaatagcg ccgtcgacca tcatcatcat catcattgaa agctgaattc c 711

<210> 91
<211> 930
<212> DNA
<213> Artificial

<220>
<223> AD1D4-D235-I10-tripB

<400> 91
atgggatcca tggcccaggt ggcatttaca ccctacgccc cggagcccgg gagcacatgc 60

cggctcagag aatactatga ccagacagct cagatgtgct gcagcaaatg ctcgccgggc 120

caacatgcaa aagtcttctg taccaagacc tcggacaccg tgtgtgactc ctgtgaggac 180

agcacataca cccagctctg gaactgggtt cccgagtgct tgagctgtgg ctcccgctgt 240

agctctgacc aggtggaaac tcaagcctgc actcgggaac agaaccgcat ctgcacctgc 300

aggcccggct ggtactgcgc gctgagcaag caggaggggt gccggctgtg cgcgccgctg 360

cgcaagtgcc gcccgggctt cggcgtggcc agaccaggaa ctgaaacatc agacgtggtg 420

tgcaagccct gtgccccggg gacgttctcc aacacgactt catccacgga tatttgcagg 480

ccccaccaga tctgtaacgt ggtggccatc cctgggaatg caagcatgga tgcagtctgc 540

acgtccacgt cccccacccg gagtatggcc ccaggggcag tacacttacc ccagccagtg 600

tccacacgat cccaacacac gcagccaact ccagaaccca gcactgctcc aagcacctcc 660

ttcctgctcc caatgggccc cagcccccca gctgaaggga gcactggcga cggtaccatt 720

gtaaatgcca agaaagatgt tgtgaacaca aagatgtttg aggagctcaa gagccgtctg 780

gacaccctgg cccaggaggt ggccctgctg aaggagcagc aggccctgca gacggtctcc 840

ctgaagggtc tagaacaaaa actcatctca gaagaggatc tgaatagcgc cgtcgaccat 900

catcatcatc atcattgaaa gctgaattcc 930

<210> 92
<211> 31
<212> DNA
<213> Artificial

<220>
<223> pKpnI-V17

<400> 92
gacggtaccg ttgtgaacac aaagatgttt g 31

<210> 93
<211> 35
<212> DNA
<213> Artificial

<220>
<223> pBAD6H

<400> 93
ggctcggaat tcaatgatga tgatgatgat ggtcg 35

<210> 94
<211> 762
<212> DNA
<213> Artificial

<220>
<223> AD1D4-GSS-V17-tripB

<400> 94
atgggatcca tggcccaggt ggcatttaca ccctacgccc cggagcccgg gagcacatgc 60

cggctcagag aatactatga ccagacagct cagatgtgct gcagcaaatg ctcgccgggc 120

caacatgcaa aagtcttctg taccaagacc tcggacaccg tgtgtgactc ctgtgaggac 180

agcacataca cccagctctg gaactgggtt cccgagtgct tgagctgtgg ctcccgctgt 240

agctctgacc aggtggaaac tcaagcctgc actcgggaac agaaccgcat ctgcacctgc 300

aggcccggct ggtactgcgc gctgagcaag caggaggggt gccggctgtg cgcgccgctg 360

cgcaagtgcc gcccgggctt cggcgtggcc agaccaggaa ctgaaacatc agacgtggtg 420

tgcaagccct gtgccccggg gacgttctcc aacacgactt catccacgga tatttgcagg 480

ccccaccaga tctgtaacgt ggtggccatc cctgggaatg caagcatgga tgcagtctgc 540

acgtccacgt cctccggttc ctccggtacc gttgtgaaca caaagatgtt tgaggagctc 600

aagagccgtc tggacaccct ggcccaggag gtggccctgc tgaaggagca gcaggccctg 660

cagacggtct ccctgaaggg tctagaacaa aaactcatct cagaagagga tctgaatagc 720

gccgtcgacc atcatcatca tcatcattga aagctgaatt cc 762

<210> 95
<211> 909
<212> DNA
<213> Artificial

<220>
<223> AD1D4-D235-V17-tripB

<400> 95
atgggatcca tggcccaggt ggcatttaca ccctacgccc cggagcccgg gagcacatgc 60

cggctcagag aatactatga ccagacagct cagatgtgct gcagcaaatg ctcgccgggc 120

caacatgcaa aagtcttctg taccaagacc tcggacaccg tgtgtgactc ctgtgaggac 180

agcacataca cccagctctg gaactgggtt cccgagtgct tgagctgtgg ctcccgctgt 240

agctctgacc aggtggaaac tcaagcctgc actcgggaac agaaccgcat ctgcacctgc 300

aggcccggct ggtactgcgc gctgagcaag caggaggggt gccggctgtg cgcgccgctg 360

cgcaagtgcc gcccgggctt cggcgtggcc agaccaggaa ctgaaacatc agacgtggtg 420

tgcaagccct gtgccccggg gacgttctcc aacacgactt catccacgga tatttgcagg 480

ccccaccaga tctgtaacgt ggtggccatc cctgggaatg caagcatgga tgcagtctgc 540

acgtccacgt cccccacccg gagtatggcc ccaggggcag tacacttacc ccagccagtg 600

tccacacgat cccaacacac gcagccaact ccagaaccca gcactgctcc aagcacctcc 660

ttcctgctcc caatgggccc cagcccccca gctgaaggga gcactggcga cggtaccgtt 720

gtgaacacaa agatgtttga ggagctcaag agccgtctgg acaccctggc ccaggaggtg 780

gccctgctga aggagcagca ggccctgcag acggtctccc tgaagggtct agaacaaaaa 840

ctcatctcag aagaggatct gaatagcgcc gtcgaccatc atcatcatca tcattgaaag 900

ctgaattcc 909

<210> 96
<211> 181
<212> PRT
<213> Homo sapiens

<400> 96

Glu Pro Pro Thr Gln Lys Pro Lys Lys Ile Val Asn Ala Lys Lys Asp
1               5                   10                  15

Val Val Asn Thr Lys Met Phe Glu Glu Leu Lys Ser Arg Leu Asp Thr
            20                  25                  30

Leu Ala Gln Glu Val Ala Leu Leu Lys Glu Gln Gln Ala Leu Gln Thr
        35                  40                  45

Val Cys Leu Lys Gly Thr Lys Val His Met Lys Cys Phe Leu Ala Phe
    50                  55                  60

Thr Gln Thr Lys Thr Phe His Glu Ala Ser Glu Asp Cys Ile Ser Arg
65                  70                  75                  80

Gly Gly Thr Leu Ser Thr Pro Gln Thr Gly Ser Glu Asn Asp Ala Leu
                85                  90                  95

Tyr Glu Tyr Leu Arg Gln Ser Val Gly Asn Glu Ala Glu Ile Trp Leu
            100                 105                 110

Gly Leu Asn Asp Met Ala Ala Glu Gly Thr Trp Val Asp Met Thr Gly
        115                 120                 125

Ala Arg Ile Ala Tyr Lys Asn Trp Glu Thr Glu Ile Thr Ala Gln Pro
    130                 135                 140

Asp Gly Gly Lys Thr Glu Asn Cys Ala Val Leu Ser Gly Ala Ala Asn
145                 150                 155                 160

Gly Lys Trp Phe Asp Lys Arg Cys Arg Asp Gln Leu Pro Tyr Ile Cys
                165                 170                 175

Gln Phe Gly Ile Val
            180

<210> 97
<211> 137
<212> PRT
<213> Homo sapiens

<400> 97

Ala Leu Gln Thr Val Cys Leu Lys Gly Thr Lys Val His Met Lys Cys
1               5                   10                  15

Phe Leu Ala Phe Thr Gln Thr Lys Thr Phe His Glu Ala Ser Glu Asp
            20                  25                  30

Cys Ile Ser Arg Gly Gly Thr Leu Ser Thr Pro Gln Thr Gly Ser Glu
        35                  40                  45

Asn Asp Ala Leu Tyr Glu Tyr Leu Arg Gln Ser Val Gly Asn Glu Ala
    50                  55                  60

Glu Ile Trp Leu Gly Leu Asn Asp Met Ala Ala Glu Gly Thr Trp Val
65                  70                  75                  80

Asp Met Thr Gly Ala Arg Ile Ala Tyr Lys Asn Trp Glu Thr Glu Ile
                85                  90                  95

Thr Ala Gln Pro Asp Gly Gly Lys Thr Glu Asn Cys Ala Val Leu Ser
            100                 105                 110

Gly Ala Ala Asn Gly Lys Trp Phe Asp Lys Arg Cys Arg Asp Gln Leu

115                    120                    125

Pro Tyr Ile Cys Gln Phe Gly Ile Val
    130                 135

<210> 98
<211> 102
<212> DNA
<213> Artificial

<220>
<223> TN-lib3-tprev

<220>
<221> misc_feature
<222> (22)..(23)
<223> randomised

<220>
<221> misc_feature
<222> (25)..(26)
<223> randomised

<220>
<221> misc_feature
<222> (28)..(29)
<223> randomised

<220>
<221> misc_feature
<222> (31)..(32)
<223> randomised

<220>
<221> misc_feature
<222> (34)..(35)
<223> randomised

<220>
<221> misc_feature
<222> (37)..(38)
<223> randomised

<220>
<221> misc_feature
<222> (40)..(41)
<223> randomised

<400> 98
gagatctggc tgggcctcaa cnnsnnsnns nnsnnsnnsn nstgggtgga catgaccggt 60

acccgcatcg cctacaagaa ctgggagact gagatcaccg cg 102

<210> 99
<211> 94
<212> DNA
<213> Artificial

<220>
<223> TN-lib2-tprev

<220>
<221> misc_feature

<222>   (17)..(17)
<223>   randomised

<220>
<221>   misc_feature
<222>   (18)..(18)
<223>   randomised

<220>
<221>   misc_feature
<222>   (20)..(21)
<223>   randomised

<220>
<221>   misc_feature
<222>   (23)..(24)
<223>   randomised

<220>
<221>   misc_feature
<222>   (29)..(29)
<223>   randomised

<220>
<221>   misc_feature
<222>   (30)..(30)
<223>   randomised

<220>
<221>   misc_feature
<222>   (32)..(33)
<223>   randomised

<400>   99
gctgggcctc aacgacnnsn nsnnsgagnn snnstgggtg gacatgaccg gtacccgcat      60

cgcctacaag aactgggaga ctgagatcac cgcg                                  94


<210>   100
<211>   108
<212>   DNA
<213>   Artificial

<220>
<223>   TN-lib3-tpfo


<220>
<221>   misc_feature
<222>   (63)..(64)
<223>   randomised

<220>
<221>   misc_feature
<222>   (66)..(67)
<223>   randomised

<220>
<221>   misc_feature
<222>   (69)..(70)
<223>   randomised

<220>
<221>   misc_feature
<222>   (72)..(73)
<223>   randomised

<220>
<221> misc_feature
<222> (75)..(76)
<223> randomised

<220>
<221> misc_feature
<222> (78)..(79)
<223> randomised

<400> 100
cgcggcagcg cttgtcgaac cacttgccgt tggccgcgcc tgacaggacc gcgcagttct 60

csnnsnnsnn snnsnnsnna tcgggttgcg cggtgatctc agtctccc 108


<210> 101
<211> 102
<212> DNA
<213> Artificial

<220>
<223> TN-lib2-tpfo


<220>
<221> misc_feature
<222> (63)..(64)
<223> randomised

<220>
<221> misc_feature
<222> (66)..(67)
<223> randomised

<220>
<221> misc_feature
<222> (69)..(70)
<223> randomised

<220>
<221> misc_feature
<222> (72)..(73)
<223> randomised

<400> 101
cgcggcagcg cttgtcgaac cacttgccgt tggccgcgcc tgacaggacc gcgcagttct 60

csnnsnnsnn snnatcgggt tgcgcggtga tctcagtctc cc 102


<210> 102
<211> 137
<212> PRT
<213> Artificial

<220>
<223> TN3-2

<400> 102

Ala Leu Gln Thr Val Cys Leu Lys Gly Thr Lys Val His Met Lys Cys
1               5                   10                  15


Phe Leu Ala Phe Thr Gln Thr Lys Thr Phe His Glu Ala Ser Glu Asp
            20                  25                  30

Cys Ile Ser Arg Gly Gly Thr Leu Ser Thr Pro Gln Thr Gly Ser Glu
        35                  40                  45

Asn Asp Ala Leu Tyr Glu Tyr Leu Arg Gln Ser Val Gly Asn Glu Ala
    50                  55                  60

Glu Ile Trp Leu Gly Leu Asn Lys Val Arg Ser Arg Tyr Phe Trp Met
65                  70                  75                  80

Asp Met Thr Gly Thr Arg Ile Ala Tyr Lys Asn Trp Glu Thr Glu Ile
                85                  90                  95

Thr Ala Gln Pro Asp Pro Arg His Thr Glu Asn Cys Ala Val Leu Ser
            100                 105                 110

Gly Ala Ala Asn Gly Lys Trp Phe Asp Lys Arg Cys Arg Asp Gln Leu
        115                 120                 125

Pro Tyr Ile Cys Gln Phe Gly Ile Val
    130                 135

<210>  103
<211>  137
<212>  PRT
<213>  Artificial

<220>
<223>  TN3-2-B

<400>  103

Ala Leu Gln Thr Val Cys Leu Lys Gly Thr Lys Val His Met Lys Cys
1               5                   10                  15

Phe Leu Ala Phe Thr Gln Thr Lys Thr Phe His Glu Ala Ser Glu Asp
            20                  25                  30

Cys Ile Ser Arg Gly Gly Thr Leu Ser Thr Pro Gln Thr Gly Ser Glu
        35                  40                  45

Asn Asp Ala Leu Tyr Glu Tyr Leu Arg Gln Ser Val Gly Asn Glu Ala
    50                  55                  60

Glu Ile Trp Leu Gly Leu Asn Lys Val Arg Ser Arg Tyr Phe Trp Met
65                  70                  75                  80

Asp Met Thr Gly Thr Arg Ile Ala Tyr Lys Asn Trp Glu Thr Glu Ile
                85                  90                  95

Thr Ala Gln Pro Asp Pro Thr Asn Asn Glu Asn Cys Ala Val Leu Ser
            100                 105                 110

Gly Ala Ala Asn Gly Lys Trp Phe Gly Lys Arg Cys Arg Asp Gln Leu

115                 120                 125

Pro Tyr Ile Cys Gln Phe Gly Ile Val
    130                 135

<210>  104
<211>  137
<212>  PRT
<213>  Artificial

<220>
<223>  TN3-2-C

<400>  104

Ala Leu Gln Thr Val Cys Leu Lys Gly Thr Lys Val His Met Lys Cys
1               5                   10                  15

Phe Leu Ala Phe Thr Gln Thr Lys Thr Phe His Glu Ala Ser Glu Asp
            20                  25                  30

Cys Ile Ser Arg Gly Gly Thr Leu Ser Thr Pro Gln Thr Gly Ser Glu
        35                  40                  45

Asn Asp Ala Leu Tyr Glu Tyr Leu Arg Gln Ser Val Gly Asn Glu Ala
    50                  55                  60

Glu Ile Trp Leu Gly Leu Asn Lys Val Arg Ser Arg Tyr Phe Trp Val
65                  70                  75                  80

Asp Met Thr Gly Thr Arg Ile Ala Tyr Lys Asn Trp Glu Thr Glu Ile
                85                  90                  95

Thr Ala Gln Pro Asp Pro Thr Asn Arg Glu Asn Cys Ala Val Leu Ser
            100                 105                 110

Gly Ala Ala Asn Gly Lys Trp Phe Asp Lys Arg Cys Arg Asp Gln Leu
        115                 120                 125

Pro Tyr Ile Cys Gln Phe Gly Ile Val
    130                 135

<210>  105
<211>  137
<212>  PRT
<213>  Artificial

<220>
<223>  TN3-2-D

<400>  105

Ala Leu Gln Thr Val Cys Leu Lys Gly Thr Lys Val His Met Lys Cys
1               5                   10                  15

Phe Leu Ala Phe Thr Gln Thr Lys Thr Phe His Glu Ala Ser Glu Asp

            20                  25                  30

Cys Ile Ser Arg Gly Gly Thr Leu Ser Thr Pro Gln Thr Gly Ser Glu
        35                  40                  45

Asn Asp Ala Leu Tyr Glu Tyr Leu Arg Gln Ser Val Gly Asn Glu Ala
    50                  55                  60

Glu Ile Trp Leu Gly Leu Asn Lys Val Arg Ser Arg Tyr Phe Trp Ile
65                  70                  75                  80

Asp Met Thr Gly Thr Arg Ile Ala Tyr Lys Asn Trp Glu Thr Glu Ile
                85                  90                  95

Thr Ala Gln Pro Asp Pro Asn Asn Arg Glu Asn Cys Ala Val Leu Ser
            100                 105                 110

Gly Ala Ala Asn Gly Lys Trp Phe Gly Lys Arg Cys Arg Asp Gln Leu
        115                 120                 125

Pro Tyr Ile Cys Gln Phe Gly Ile Val
    130                 135

<210> 106
<211> 181
<212> PRT
<213> Artificial

<220>
<223> TN-2-B

<400> 106

Glu Pro Pro Thr Gln Lys Pro Lys Lys Ile Val Asn Ala Lys Lys Asp
1               5                   10                  15

Val Val Asn Thr Lys Met Phe Glu Glu Leu Lys Ser Arg Leu Asp Thr
            20                  25                  30

Leu Ala Gln Glu Val Ala Leu Leu Lys Glu Gln Gln Ala Leu Gln Thr
        35                  40                  45

Val Cys Leu Lys Gly Thr Lys Val His Met Lys Cys Phe Leu Ala Phe
    50                  55                  60

Thr Gln Thr Lys Thr Phe His Glu Ala Ser Glu Asp Cys Ile Ser Arg
65                  70                  75                  80

Gly Gly Thr Leu Ser Thr Pro Gln Thr Gly Ser Glu Asn Asp Ala Leu
                85                  90                  95

Tyr Glu Tyr Leu Arg Gln Ser Val Gly Asn Glu Ala Glu Ile Trp Leu
            100                 105                 110

Gly Leu Asn Lys Val Arg Ser Arg Tyr Phe Trp Met Asp Met Thr Gly
        115                 120                 125

Thr Arg Ile Ala Tyr Lys Asn Trp Glu Thr Glu Ile Thr Ala Gln Pro
    130                 135                 140

Asp Pro Thr Asn Asn Glu Asn Cys Ala Val Leu Ser Gly Ala Ala Asn
145                 150                 155                 160

Gly Lys Trp Phe Gly Lys Arg Cys Arg Asp Gln Leu Pro Tyr Ile Cys
                165                 170                 175

Gln Phe Gly Ile Val
            180

<210>  107
<211>  181
<212>  PRT
<213>  Artificial

<220>
<223>  TN-2-D

<400>  107

Glu Pro Pro Thr Gln Lys Pro Lys Lys Ile Val Asn Ala Lys Lys Asp
1               5                   10                  15

Val Val Asn Thr Lys Met Phe Glu Glu Leu Lys Ser Arg Leu Asp Thr
            20                  25                  30

Leu Ala Gln Glu Val Ala Leu Leu Lys Glu Gln Gln Ala Leu Gln Thr
        35                  40                  45

Val Cys Leu Lys Gly Thr Lys Val His Met Lys Cys Phe Leu Ala Phe
    50                  55                  60

Thr Gln Thr Lys Thr Phe His Glu Ala Ser Glu Asp Cys Ile Ser Arg
65                  70                  75                  80

Gly Gly Thr Leu Ser Thr Pro Gln Thr Gly Ser Glu Asn Asp Ala Leu
                85                  90                  95

Tyr Glu Tyr Leu Arg Gln Ser Val Gly Asn Glu Ala Glu Ile Trp Leu
            100                 105                 110

Gly Leu Asn Lys Val Arg Ser Arg Tyr Phe Trp Ile Asp Met Thr Gly
        115                 120                 125

Thr Arg Ile Ala Tyr Lys Asn Trp Glu Thr Glu Ile Thr Ala Gln Pro
    130                 135                 140

Asp Pro Asn Asn Arg Glu Asn Cys Ala Val Leu Ser Gly Ala Ala Asn

145                 150                 155                 160

Gly Lys Trp Phe Gly Lys Arg Cys Arg Asp Gln Leu Pro Tyr Ile Cys
                165                 170                 175

Gln Phe Gly Ile Val
            180

<210>  108
<211>  181
<212>  PRT
<213>  Artificial

<220>
<223>  TN-2-C

<400>  108

Glu Pro Pro Thr Gln Lys Pro Lys Lys Ile Val Asn Ala Lys Lys Asp
1               5                   10                  15

Val Val Asn Thr Lys Met Phe Glu Glu Leu Lys Ser Arg Leu Asp Thr
            20                  25                  30

Leu Ala Gln Glu Val Ala Leu Leu Lys Glu Gln Gln Ala Leu Gln Thr
        35                  40                  45

Val Cys Leu Lys Gly Thr Lys Val His Met Lys Cys Phe Leu Ala Phe
    50                  55                  60

Thr Gln Thr Lys Thr Phe His Glu Ala Ser Glu Asp Cys Ile Ser Arg
65                  70                  75                  80

Gly Gly Thr Leu Ser Thr Pro Gln Thr Gly Ser Glu Asn Asp Ala Leu
                85                  90                  95

Tyr Glu Tyr Leu Arg Gln Ser Val Gly Asn Glu Ala Glu Ile Trp Leu
            100                 105                 110

Gly Leu Asn Lys Val Arg Ser Arg Tyr Phe Trp Val Asp Met Thr Gly
        115                 120                 125

Thr Arg Ile Ala Tyr Lys Asn Trp Glu Thr Glu Ile Thr Ala Gln Pro
    130                 135                 140

Asp Pro Thr Asn Arg Glu Asn Cys Ala Val Leu Ser Gly Ala Ala Asn
145                 150                 155                 160

Gly Lys Trp Phe Asp Lys Arg Cys Arg Asp Gln Leu Pro Tyr Ile Cys
                165                 170                 175

Gln Phe Gly Ile Val
            180

<210> 109
<211> 256
<212> PRT
<213> Artificial

<220>
<223> AD1D4-GSS-I10

<400> 109

Met Gly Ser Met Ala Gln Val Ala Phe Thr Pro Tyr Ala Pro Glu Pro
1               5                   10                  15

Gly Ser Thr Cys Arg Leu Arg Glu Tyr Tyr Asp Gln Thr Ala Gln Met
            20                  25                  30

Cys Cys Ser Lys Cys Ser Pro Gly Gln His Ala Lys Val Phe Cys Thr
        35                  40                  45

Lys Thr Ser Asp Thr Val Cys Asp Ser Cys Glu Asp Ser Thr Tyr Thr
    50                  55                  60

Gln Leu Trp Asn Trp Val Pro Glu Cys Leu Ser Cys Gly Ser Arg Cys
65                  70                  75                  80

Ser Ser Asp Gln Val Glu Thr Gln Ala Cys Thr Arg Glu Gln Asn Arg
                85                  90                  95

Ile Cys Thr Cys Arg Pro Gly Trp Tyr Cys Ala Leu Ser Lys Gln Glu
            100                 105                 110

Gly Cys Arg Leu Cys Ala Pro Leu Arg Lys Cys Arg Pro Gly Phe Gly
        115                 120                 125

Val Ala Arg Pro Gly Thr Glu Thr Ser Asp Val Val Cys Lys Pro Cys
    130                 135                 140

Ala Pro Gly Thr Phe Ser Asn Thr Thr Ser Ser Thr Asp Ile Cys Arg
145                 150                 155                 160

Pro His Gln Ile Cys Asn Val Val Ala Ile Pro Gly Asn Ala Ser Met
                165                 170                 175

Asp Ala Val Cys Thr Ser Thr Ser Ser Gly Ser Ser Gly Thr Ile Val
            180                 185                 190

Asn Ala Lys Lys Asp Val Val Asn Thr Lys Met Phe Glu Glu Leu Lys
        195                 200                 205

Ser Arg Leu Asp Thr Leu Ala Gln Glu Val Ala Leu Leu Lys Glu Gln
    210                 215                 220

Gln Ala Leu Gln Thr Val Ser Leu Lys Gly Leu Glu Gln Lys Leu Ile

225                     230                     235                     240

Ser Glu Glu Asp Leu Asn Ser Ala Val Asp His His His His His His
                245                 250                 255

## Description

### Field of the invention

The present invention pertains to the provision of trimeric binding proteins which bind to trimeric cytokines. In particular there is provided trimeric binders that bind in a manner such that upon binding, all receptor binding sites of the trimeric cytokine are substantially blocked, and hence the potential biological activity of the trimeric cytokine is suppressed. In one aspect the invention relates to trimeric binders capable of binding to cytokines of the Tumour necrosis factor ligand superfamily, including tumour necrosis factor (TNF).

### Background of the invention and prior art

Cytokines are small secreted polypeptides from higher eukaryotes which are responsible for intercellular signal transduction and which affect the growth, division and functions of other cells. They are potent, pleiotropic polypeptides that, e.g. via corresponding receptors, act as local or systemic intercellular regulatory factors, and therefore play crucial roles in many biologic processes, such as immunity, inflammation, and hematopoiesis (formation and development of blood cells in the bone marrow). Cytokines are produced by diverse cell types including fibroblasts, endothelial cells, macrophages/monocytes, and lymphocytes. To date, a large number of cytokines have been identified, including interferons, tumour necrosis factors, interleukins and lymphokines. They differ from classical hormones in that they are produced by a number of tissues or cell types rather than by specialized glands.

The modulation of cytokine-receptor interactions can be a useful mechanism for therapeutic intervention in various diseases and pathologies. Soluble binding proteins, which can interact with cytokines, can potentially sequester the cytokine away from the receptor, thereby reducing or preventing the activation of that particular receptor pathway.

Certain cytokines occur as multi-subunit complexes containing multiple copies of the same subunit. Macrophage migration inhibitory factor (MIF) and proteins within the tumour necrosis factor ligand super family (TNFLSF), such as Tumour necrosis factor (TNF; TNFLSF member 2) and lymphotoxin alpha (LT-alpha; TNFLSF member 1), occur as homotrimers formed by three identical subunits. Many of these trimeric cytokines are known to be involved in signal transduction and to act as regulatory factors, and to be involved in many different types of diseases and medical indications.

The trimeric cytokines of the TNF ligand super family are known to control and orchestrate the immune and inflammatory responses at several levels. It is also known that TNF ligand super family members are associated with several disease conditions, such as acute or chronic inflammatory conditions. Presently, the TNF ligand super family has at least 17 recognised ligands, including LTA (lymphotoxin alpha; TNFLSF member 1), TNF (tumor necrosis factor; TNFLSF member 2), LTB (lymphotoxin beta; TNFLSF member 3), OX-40L TNFLS member 4); CD40L (TNFLS member 5); FasL (TNFLS member 6); CD27L (TNFLS member 7); CD30L (TNFLS member 8); 4-1 BB-L (TNFLS member 9); TRAIL (TNFLS member 10); RANKL (TNFLS member 11); TWEAK (TNFLS member 12); APRIL (TNFLS member 13); BAFF (TNFLS member 13B); LIGHT (TNFLS member 14); VEGI (TNFLS member 15); and GITRL (TNFLS member 18).

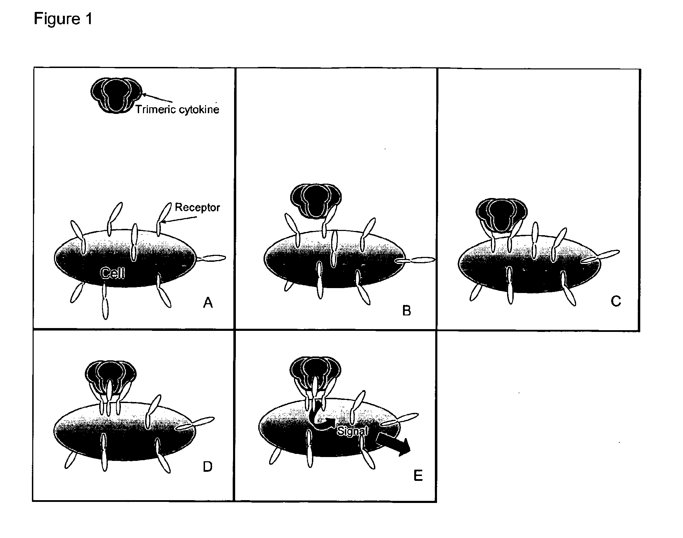

Evidence exist that the signalling unit of the trimeric cytokines of the TNF ligand super family and their corresponding receptors, is in the form of three receptors and three ligands assembled as a hexameric complex in which a single cytokine trimer binds to three receptor molecules (Bodmer et al. 2002, TRENDS in Biochemical Sciences 27(1), pp. 19-26). Before becoming part of this hexameric complex, the receptors exist in monomeric form. This general mechanism is illustrated in Figure 1.

TNF (TNFLSF member 2) is one of the principal mediators of the immune and inflammatory response, and it is e.g. known to have an important role in the pathogenesis of rheumatoid arthritis, which is a common autoimmune inflammatory disease that affects approximately 0.5-1 % of the human population. Additionally, TNF is also known to be involved in the pathogenesis of a wide range of disease states, including endotoxin shock, cerebral malaria and graft-versus-host reaction. TNF is produced by a number of cell types, mainly by activated macrophages. The soluble form of TNF consists of three identical 17 kD protein subunits, whereas the membrane bound form consist of three identical 26 kD subunits. TNF is also previously known as "TNF-alpha" and "cachectin".

TWEAK (TNFLSF member 12) was recently found to be a direct and strong inducer of angiogenesis (formation and growth of blood vessels) as it was observed that picomolar concentrations of TWEAK promote proliferation of normal endothelial cells and that TWEAK induces angiogenesis in an *in vivo* rat cornea model (Lynch et al., 1999, The Journal of Biological Chemistry, 274(13) pp. 8455-8459). In particular angiogenesis is essential for the growth and persistence of solid tumours and their metastases, and it is also known to be involved in other pathological conditions such as diabetic retinopathy, psoriasis, contact dermatitis and restenosis.

In US20020037852A1 the trimeric cytokine BAFF (TNFLS member 13B) was described to be expressed by T cells and dendritic cells for the purpose of B-cell co-stimulation and consequently may play an important role in the control of B cell function. It is suggested therein that BAFF and its receptor may have anti-cancer and immunoregulatory applications as well as uses for the treatment of immunosuppressive disorders such as HIV.

APRIL (TNFLS member 13) is a trimeric cytokine that binds to the receptor TNFRSF13B and to TNFRSF17. It has recently been shown that the addition of recombinant APRIL to various tumor cells stimulates their proliferation and hence APRIL may be implicated in the regulation of tumor cell growth (Hahne M., et al, 1998; J. Exp. Med. 188:1185-1190). It has also been suggested that APRIL may be involved in monocyte/macrophage-mediated immunological processes.

Furthermore, cytokines within the TNF super family have also been described to be involved in hereditary diseases such as hyper IgM syndrome, type I autoimmune lymphoproliferative syndrome, TNF-R1-associated periodic fever syndrome, hypohidrotic ectodermal dysplasia and familial expansile osteolysis.

Several attempts have been made in order to find and develop suitable antagonists and binders for trimeric cytokines which are capable of binding to specific trimeric cytokines and thereby preventing the trimeric cytokines from binding to e.g. cell membrane bound receptors and hence suppressing the activation of that particular receptor pathway.

One example is antagonists against the trimeric cytokine TNF (TNFLSF member 2). Presently, two types of TNF antagonists are commercially available, namely Infliximab (Remicade) and Eternarcept (Enbrel) which have both received marketing authorization in the United States and Europe for treatment of rheumatoid arthritis. The two products have also been shown to be effective for the treatment of psoriasis and Chrohn's disease. Infliximab is a chimeric antibody with murine variable regions and human IgG1 and ? constant regions, which neutralizes the biological activity of TNF by binding to the soluble and transmembrane forms of TNF and inhibits the binding of TNF with its receptors. The structure of Infliximab is similar to that of naturally occurring antibodies. Eternacept is a fusion protein made up of the extracellular domain of the p75 TNF receptor and the hinge and Fc domains of human IgG1.

Another example of an antagonist for a trimeric cytokine is given in WO 00/42073 where a monoclonal antibody against the trimeric cytokine TWEAK (TNFLS member 12) is disclosed. It is mentioned that the antibody can block the development of Graft-Versus-Host Disease.

However, a common technical problem that is encountered with the presently known binders and inhibitor products for trimeric cytokines, is that upon formation of a 1:1 complex of the trimeric cytokine and the binder or inhibitor product, not all three receptor binding sites of the trimeric cytokines are effectively blocked, as at least one or two of the three receptor binding sites are left open. The open cytokine receptor binding site(s) may associate with a corresponding cell surface cytokine receptor and thereby, due to high local concentration of the receptors on the cell surface, initiate the further recruitment of cytokine receptors and mediate receptor signalling.

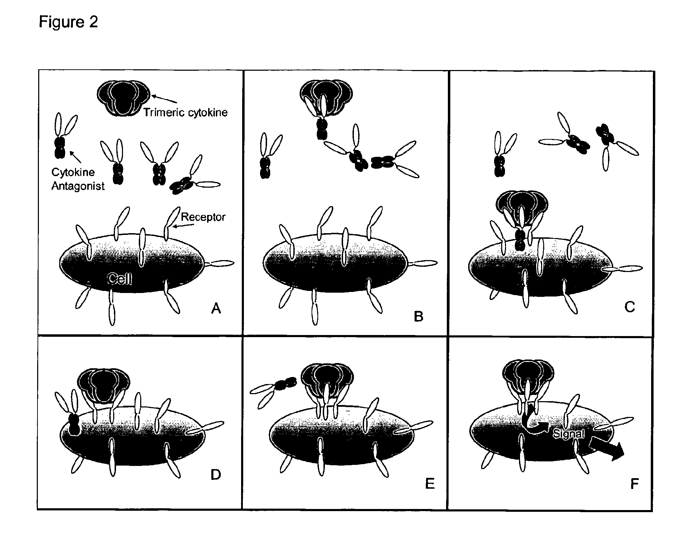

The problem is clearly illustrated by the TNF binder Eternacept, which is a bivalent molecule that forms a 1:1 complex with the TNF trimer. When Eternacept binds to TNF, only two out off three possible receptor binding sites are occupied by the Eternacept molecule, and the third receptor binding site is left open. This implies that the open TNF receptor binding site may associate with a cell surface TNF receptor and thereby, due to high local concentration of the receptors on the cell surface, initiate the further recruitment of TNF receptors and by that signal transduction. This problem is illustrated in Figure 2.

The same problem is seen with the TNF binder Infliximab. Each Infliximab molecule is only capable of binding one receptor binding site (subunit) of a given TNF molecule, hence leaving out two open receptor binding sites. These open receptor binding sites may subsequently associate with corresponding free receptors, resulting in further recruitment of TNF receptors and receptor signalling.

It has now been found by the present inventors, that the above technical problems may be overcome by the provision of a fusion protein (protomer) which is constructed as a fusion of a binding unit (binder) for trimeric cytokines to a trimerising domain. The fusion protein of the present invention is, as a trimeric protein, capable of forming a 1:1 complex with a trimeric cytokine and preferably at the same time effectively binding all three receptor binding sites of the trimeric cytokines, whereby no receptor binding sites are left open, and is therefore able to inhibit docking of the cytokine to the cell surface. Thus, there is now provided a fusion protein which has the advantage of being capable of more effectively inhibiting and neutralising the biological activity of trimeric cytokines as compared to presently known trimeric cytokine antagonists. A further advantage of the fusion protein according to the invention is that when applied as a medicament, less amount of the fusion protein is required in order to obtain a therapeutic effect, as compared to the presently know binders for trimeric cytokines.

### Summary of the invention

Accordingly, the invention relates in a first aspect to a trimeric polypeptide comprising three monomers, each of said monomers comprising a specific binding member capable of binding a trimeric cytokine, and each of said monomers comprising a trimerising domain.

In a further aspect there is provided a pharmaceutical composition comprising the trimeric polypeptide according to the invention.

In a still further aspect, the invention pertains to a method of treating a subject having a pathology mediated by a trimeric cytokine such as tumour necrosis factor, by administering an effective amount of the trimeric polypeptide according to the invention to the subject,

Finally, there is provided a method for the preparation of the trimeric polypeptide according to invention, and an assay method for detecting a trimeric polypeptide cytokine in a sample.

### Detailed disclosure of the invention

In one aspect, the present invention provides trimeric binders which are capable of binding to trimeric cytokines, preferably in a manner such that upon binding, all receptor binding sites of the trimeric cytokine are substantially blocked and/or neutralised, and hence the potential biological activity of the trimeric cytokine is suppressed or neutralised. Accordingly, there is provided a trimeric polypeptide comprising three monomers, wherein each of the monomers comprise a specific binding member which is capable of binding a trimeric cytokine, and wherein each of the monomers comprise a trimerising domain.

In the present context, the term "trimeric cytokine" refers to small proteins and fragments thereof, which are produced and secreted by a cell, and which elicit a specific response in a cell which has a receptor for that cytokine, e.g. by affecting the growth, division and/or function of the cell. The term "trimeric" is used herein to describe that the cytokines according to the invention occur as multi-subunit complexes containing three subunits, preferably three identical subunits (homotrimer). Typical examples of such trimeric cytokines include Macrophage migration inhibitory factor (MIF) and cytokines within the tumour necrosis factor ligand super family (TNFLSF). Presently, as mentioned above, the TNF ligand super family has at least 17 recognised ligands which all share a conserved trimeric C-terminal domain known as the "TNF homology domain" (THD), which is responsible for receptor binding. The sequence identity of this trimeric domain is approximately 20-30% between TNF family members. The THD is a 150 amino acid long sequence containing a conserved framework of aromatic and hydrophobic residues which is illustrated in Figure 2 in Bodmer et al. 2002, TRENDS in Biochemical Sciences 27(1), pp. 19-26. Accordingly, in the present context a "member of the Tumor necrosis factor ligand superfamily" is intended to mean a trimeric protein comprising the TNF homology domain, THD. Presently known trimeric cytokines within the TNF ligand super family are listed below in Table 1, together with synonyms and their corresponding Genbank ID (GenBank, National Center for Biotecnology Information; http://www.ncbi.nlm.nih.gov/Genbank). The nomenclature for the TNF ligand superfamily as used herein follows the official TNF superfamily (TNFSF) nomenclature which may be found at http://www.gene.uci.ac.uk/nomenciature/genefamiiy/tnftop.htm!.

**TABEL 1: TNF ligand superfamily**

| Ligand symbol | Genbank ID | Synonyms |
|---|---|---|
| LTA | X01393 | TNFSF1, TNFB, LT |
| TNF | X02910 | TNFSF2, TNF-alpha, DIF |
| LTB | L11016 | TNFSF3. TNFC, p33 |
| TNFSF4 | D90224 | OX-40L, gp34, TXGP1 |
| TNFSF5 | X67878 | CD40LG, IMD3, HIGM1, CD40L, hCD40L, TRAP, CD154, gp39 |
| TNFSF6 | U11821 | FasL, APT1LG1 |
| TNFSF7 | L08096 | CD70, CD27L, CD27LG |
| TNFSF8 | L09753 | CD30LG |
| TNFSF9 | U03398 | 4-1BB-L |
| TNFSF10 | U37518 | TRAIL, Apo-2L, TL2 |
| TNFSF11 | AF013171 | TRANCE, RANKL, OPGL, ODF |
| TNFSF12 | AF030099 | TWEAK, DR3LG, AP03L |
| TNFSF13 | NM_003808 | APRIL |
| TNFSF13B | AF136293 | BAFF, THANK, BLYS, TALL-1, TALL1, TNFSF20 |
| TNFSF14 | AF036581 | LIGHT, LTg, HVEM-L |
| TNFRSF Fn14 | - | Fibroblast growth factor-inducible immediate-early response protein 14, FGF-inducible 14, Tweak-receptor, TweakR |
| TNFSF15 | AF039390 | TL1, VEGI |
| TNFSF18 | AF125303 | AITRL TL6 hGITRL |

Accordingly, in one useful embodiment of the invention the trimeric polypeptide according to the invention is capable of binding a trimeric cytokine which is a member of the Tumor necrosis factor ligand superfamily selected from LTA; TNF ;LTB; TNFSF4; TNFSF5; TNFSF6; TNFSF7; TNFSF8; TNFSF9; TNFSF10; TNFSF11; TNFSF12; TNFSF13; TNFSF13B; TNFSF14; TNFRSF Fn14; TNFSF15; and TNFSF18.

The term "specific binding member", as used herein, refers to a member of a pair of molecules which have binding specificity for one another. The members of a specific binding pair may be naturally derived or wholly or partially synthetically produced. One member of the pair of molecules has an area on its surface, or a cavity, which specifically binds to and is therefore complementary to a particular spatial and polar organisation of the other member of the pair of molecules. Thus the members of the pair have the property of binding specifically to each other. Examples of types of specific binding pairs are antigen-antibody, biotin-avidin, hormone-hormone receptor, ligand-ligand receptor, enzyme-substrate. Other examples of specific binding pairs include, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, enzyme cofactors and enzymes, enzyme inhibitors and enzymes, and the like. Furthermore, specific binding pairs can include members that are analogues or fragments of the original specific binding member.

In useful embodiments, the specific binding members capable of binding a trimeric cytokine, provides the trimeric polypeptide according to the invention with a binding affinity (Kd) for the trimeric cytokine that preferably is 1 x 10⁻⁸ M or less determined by surface plasmon resonance. In other useful embodiments, the Kd value is less than 1 x 10⁻⁹ M, 1 x 10⁻¹⁰ M, 1 x 10⁻¹¹ M, 1 x 10⁻¹² M, 1 x 10⁻¹³ M, 1 x 10⁻¹⁴ M, or even less than 1 x 10⁻¹⁵ M. In further useful embodiments, the K-off rate for the trimeric polypeptide according to the invention is less than 1 x 10⁻³ S⁻¹, such as less than 1 x 10⁻⁴ S⁻¹, including less than 1 x 10⁻⁵ S⁻¹, and even less than 1 x 10⁻⁶ S⁻¹ determined by surface plasmon resonance. The term "K-off", as used herein, is intended to refer to the off rate constant for dissociation of a specific binding member from the specific binding member/trimeric cytokine complex. The term "surface plasmon resonance", as used herein, refers to an optical phenomenon that allows for the analysis of real-time biospecific interactions by detection of alterations in protein concentrations within a biosensor matrix, for example using the BlAcore system (Pharmacia Biosensor AB, Uppsala, Sweden). For further descriptions, see Example 10.

As mentioned above, it is preferred that the trimeric cytokine binder according to the invention at least partially or fully blocks, inhibits, or neutralises a biological activity of a trimeric cytokine. As used herein, the expression "neutralises" or "neutralisation" means the inhibition of or reduction in a biological activity of a trimeric cytokine as measured *in vivo* or *in vitro.*

In useful embodiments the specific binding member is a polypeptide derived from a member of the Tumor necrosis factor receptor superfamily. Presently, at least 29 receptors within the Tumor necrosis factor superfamily are known, and listed below in Table 2 together with their synonyms and their corresponding Genbank ID (GenBank, National Center for Biotecnology Information; http://www.ncbi.nlm.nih.gov/Genbank). The nomenclature for the members of the TNF receptor superfamily as used herein follows the official TNF superfamily (TNFSF) nomenclature which may be found at http://www.gene.ucl.ac.uk/nomenclature/genefamily/tnftop.html.

**TABEL 2: TNF receptor superfamily**

| Receptor Symbol | Genbank ID | Synonyms |
|---|---|---|
| TNFRSF1A | M75866 | p55-R, CD120a, TNF-R-I p55, TNF-R, TNFR1, TNFAR, TNF-R55, p55TNFR, TNFR60 |
| TNFRSF1B | M32315 | CD120b, p75, TNF-R, TNF-R-II, TNFR80, TNFR2,TNF-R75, TNFBR, p75TNFR |
| LTBR | L04270 | TNFRSF3, TNFR2-RP, CD18, TNFR-RP, TNFCR, TNF-R-III |
| TNFRSF4 | X75962 | OX40, ACT35, TXGP1L |
| TNFRSF5 | X60592 | p50, Bp50. CD40 |
| TNFRSF6 | M67454 | FAS, CD95, APO-1, APT1 |
| TNFRSF6B | AF104419 | DcR3, M68. TR6, HGNC:15888, NHL, DKFZP434C013, KIAA1088, bK3184A7.3, C20orf41 |
| TNFRSF7 | M63928 | Tp55, S152, CD27 |
| TNFRSF8 | M83554 | Ki-1, D1S166E, CD30 |
| TNFRSF9 | L12964 | 4-1BB, CD137, ILA |
| TNFRSF10A | U90875 | DR4, Apo2, TRAILR-1 |
| TNFRSF10B | AF012628 | DR5, KILLER, TRICK2A, TRAIL-R2, TRICKB |
| TNFRSF10C | AF012536 | DcR1, TRAILR3, LIT, TRID |
| TNFRSF10D | AF029761 | DcR2, TRUNDD, TRAILR4 |
| TNFRSF11A | AF018253 | RANK |
| TNFRSF11B | U94332 | OPG, OCIF, TR1 |
| TNFRSF12 | U72763 | DR3, TRAMP, WSL-1, LARD, WSL-LR,DDR3, TR3, APO-3 |
| TNFRSF12L | - | DR3L |
| TNFRSF13B | AF023614 | TACI |
| TNFRSF13C | AF373846 | BAFFR |
| TNFRSF14 | U70321 | HVEM, ATAR, TR2, LIGHTR, HVEA |
| NGFR | M14764 | TNFRSF16, p75NTR |
| TNFRSF17 | Z29574 | BCMA, TNFRSF13 |
| TNFRSF18 | AF125304 | AITR, GITR |
| TNFRSF19 | AB040434 | TAJ-alpha, TROY, TAJ. TRADE |
| TNFRSF19L | AF319553 | FLJ14993, RELT |
| TNFRSF21 | AF068868 | DR6 |
| TNFRSF22 | - | SOBa, Tnfrh2, 2810028K06Rik |
| TNFRSF23 | - | mSOB, Tnfrh1 |

Thus, in useful embodiments the specific binding member derived from a receptor within the Tumor necrosis factor superfamily is selected from the receptors TNFRSF1A, TNFRSF1B, LTBR, TNFRSF4, TNFRSF5, TNFRSF6, TNFRSF6B, TNFRSF7, TNFRSF8, TNFRSF9, TNFRSF10A, TNFRSF10B, TNFRSF10C, TNFRSF10D, TNFRSF11A, TNFRSF11B, TNFRSF12, TNFRSF12L, TNFRSF13B, TNFRSF13C, TNFRSF14, NGFR, TNFRSF17, TNFRSF18, TNFRSF19, TNFRSF19L, TNFRSF21, TNFRSF22, and TNFRSF23. However, it is also contemplated that further useful receptors may be identified within this protein family, and hence further functional specific binding members.

In a useful embodiment, the specific binding member is derived from the TNF receptors TNFRSF1A (TNFrl, p55 receptor) and/or TNFRSF1 B (TNFrII, p75 TNF receptor). Especially the TNFRSF1 B receptor and subunits thereof has proven to be effective for specifically binding and neutralising TNF. As an example, the TNF binding protein Eternacept is a fusion protein made up of the extracellular domain of the TNFRSF1 B receptor, i.e. a subunit of the TNFRSF1 B receptor. Accordingly, in specific embodiments, the trimeric polypeptide according to invention includes a specific binding member which is a subunit or fragment of the TNFRSF1 B receptor (TNFrII), such as the TNFRSF1 B receptor fragments disclosed in US 5,712,155. Thus, in useful embodiments the specific binding member is a polypeptide comprising an amino acid sequence selected from TNFRSF1B 1-235 (SEQ ID NO:76), TNFRSF1B 1-185 (SEQ ID NO:77), TNFRSF1B 1-163 (SEQ ID NO:78) and TNFRSF1B 1-142 (SEQ ID NO:79).

In further useful embodiments, as disclosed in following examples, the specific binding member may be one or more fragments of the TNFRSF1 B receptor (TNFrII) and combinations of such fragments, such as a polypeptide comprising an amino acid sequence encoded by a DNA sequence selected from TNFRSF1 B D1 D2 (SEQ ID NO:13), TNFRSF1 B D1 D2, 1/6 (SEQ ID NO:15), TNFRSF1B D1 D2 1/4 (SEQ ID NO:17), TNFrII D1 D2, 1/3 (SEQ ID NO:19), TNFRSF1 B D1 D2, 1/2 (SEQ ID NO:21), TNFRSF1B D1D4 (SEQ ID NO:23), TNFRSF1 B D2 (SEQ ID NO:25), TNFRSF1 B D2, 1/6 (SEQ ID NO:26), TNFRSF1B D2, 1/4 (SEQ ID NO:27), TNFRSF1B D2, 1/3 (SEQ ID NO:28), TNFRSF1B D2, 1/2 (SEQ ID NO:29) and TNFRSF1B D2D4 (SEQ ID NO:30).

In accordance with the invention, the specific binding member capable of binding a trimeric cytokine may also be an antibody or an antibody fragment. In the present context, the term "antibody" is used to describe an immunoglobulin whether natural or partly or wholly synthetically produced. As antibodies can be modified in a number of ways, the term "antibody" should be construed as covering any specific binding member or substance having a binding domain with the required trimeric cytokine specificity. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. The term also covers any polypeptide or protein having a binding domain which is, or is homologous to, an antibody binding domain, e.g. antibody mimics. These can be derived from natural sources, or they may be partly or wholly synthetically produced. Examples of antibodies are the immunoglobulin isotypes and their isotypic subclasses; fragments which comprise an antigen binding domain such as Fab, scFv, Fv, dAb, Fd; and diabodies.

US 6,451,983 B2 describes antibodies for human tumour necrosis factor which are i.a. capable of neutralising the biological activity of TNF. In particular, US 6,451,983 B2 provides antibodies and fragments thereof which are capable of binding to mature human TNF (SEQ ID NO:80) within certain specific topographic regions. Accordingly, in useful embodiments of the present invention, the specific binding member is an antibody or fragment thereof which is capable of binding mature human TNF (SEQ ID NO:80) in at least one region selected from the regions consisting of amino acid residues 1-18, 1-20, 1-26, 1-30, 12-22, 22-31, 22-40, 36-45, 49-97, 49-98 , 56-79, 58-65, 69-97, 70-87, 76-90, 96-105, 105-128, 108-128, 110-127, 115-125, 117-128, 132-157, 135-155, 136-153, 138-149, 141-153 and 146-157.

US 6,451,983 B2 further provides specific monoclonal antibodies which may be useful in the present invention. Accordingly, the specific binding member capable of binding human TNF may be an antibody or antibody fragment selected from MAb 1 (ECACC 89080301), MAb 21 (ECACC 90012432), MAb 25 (ECACC 89121401), MAb 32 (ECACC 89080302), MAb 37 (ECACC 89080303), MAb 42 (ECACC 89080304), MAb 47 (ECACC 89121402), MAb 53 (ECACC 90012433) and MAb 54 (ECACC 89083103), wherein ECACC refers to European Collection of Animal Cell Cultures.

Further examples of useful antibodies include the humanised antibody D2E7 as disclosed in US 6,090,382 and the humanised antibody fragment CDP 870 as disclosed in WO/00194585.

In specific embodiments, the specific binding members of the present invention may comprise antibody analogues or artificial antibodies such as e.g. a protein having the scaffold structure of C-type lectin-like domains (CTLD) as disclosed in WO/0248189. As will be apparent from the following Examples, useful human tetranectin based CTLD antibody analogues which are capable of binding TNF may be selected from TN3-2-B (SEQ ID NO:103), TN3-2-C (SEQ ID NO:104) and TN3-2-D (SEQ ID NO:105).

An important aspect of the present invention is that the monomers of the trimeric polypeptide, in addition to the specific binding member, further comprise a trimerising domain. In the present context, the term "trimerising domain" is a peptide, a protein or part of a protein which is capable of interacting with other, similar or identical trimerising domains. The interaction is of the type that produces trimeric proteins or polypeptides. Such an interaction may be caused by covalent bonds between the components of the trimerising domains as well as by hydrogen bond forces, hydrophobic forces, van der Waals forces and salt bridges.

One example of a trimerising domain is disclosed in WO 95/31540, which describes polypeptides comprising a collectin neck region. The amino acid sequence constituting the collectin neck region may be attached to any polypeptide of choice. Trimers can then be made under appropriate conditions with three polypeptides comprising the collectin neck region amino acid sequence.

In a presently preferred embodiment, the trimerising domain is derived from tetranectin, and more specifically comprises the tetranectin trimerising structural element (hereafter termed TTSE) which is described in detail in WO 98/56906. The amino acid sequence of TTSE is shown in SEQ ID NO:81. The trimerising effect of TTSE is caused by a coiled coil structure which interacts with the coiled coil structure of two other TTSEs to form a triple alpha helical coiled coil trimer which is exceptionally stable even at relatively high temperatures. The term TTSE is also intended to embrace variants of a TTSE of a naturally occurring member of the tetranectin family of proteins, variants which have been modified in the amino acid sequence without adversely affecting, to any substantial degree, the capability of the TTSE to form alpha helical coiled coil trimers. Thus, the trimeric polypeptide according to the invention may comprise a TTSE as a trimerising domain, which comprises a sequence having at least 68% amino acid sequence identity with the sequence of SEQ ID NO SEQ ID NO:81, such as at least 75%, including at least 87%, such as at least 92%. In accordance herewith, the cystein residue No. 50 of the TTSE (SEQ ID NO:81) may advantageously be mutagenised to serine, threonine, methionine or to any other amino acid residue in order to avoid formation of an unwanted inter-chain disulphide bridge, which could lead to unwanted multimerisation.

In a further embodiment, the TTSE trimerising domain (SEQ ID NO:81) may be modified by (i) the incorporation of polyhistidine sequence and/or a cleavage site for the Blood Coagulating Factor Xₐ, (ii) replacing Cys 50 with Ser, and (iii) by including a C-terminal KGS sequence.

Several examples of different trimeric polypeptides in accordance with the invention are provided in the following in examples, in which the above TTSE trimerising domain has been applied. These includes i.a. the CTLD based TNF binding units TN-2-B (SEQ ID NO:106), TN-2-C (SEQ ID NO:108) and TN-2-D (SEQ ID NO:107), and the trimerised human TNFRII fragment AD1D4-GSS-110 (SEQ ID NO:109).

In accordance with the invention, the specific binding member may either be linked to the N- or the C-terminal amino acid residue of the trimerising domain. However, it is also envisaged that in certain embodiments it may be advantageous to link a specific binding member to both the N-terminal and the C-terminal of the trimerising domain of the monomer, and thereby providing a trimeric polypeptide comprising six specific binding members capable of binding a trimeric cytokine.

It will be appreciated that a flexible molecular linker optionally may be interposed between, and covalently join, the specific binding member and the trimerising domain. In certain embodiments, the linker is a polypeptide sequence of about 1-20 amino acid residues. The linker may be less than 10 amino acids, most preferably, 5, 4, 3, 2, or 1. It may be in certain cases that 9, 8, 7 or 6 amino acids are suitable. In useful embodiments the linker is essentially non-immunogenic, not prone to proteolytic cleavage and does not comprise amino acid residues which are known to interact with other residues (e.g. cystein residues).

The trimeric polypeptide of the present invention may be expressed in any suitable standard protein expression system by culturing a host transformed with a vector encoding the trimeric polypeptide under such conditions that the trimeric polypeptide is expressed. Preferably, the expression system is a system from which the desired protein may readily be isolated and refolded *in vitro.* As a general matter, prokaryotic expression systems are preferred since high yields of protein can be obtained and efficient purification and refolding strategies are available. Thus, it is well within the abilities and discretion of the skilled artisan, without undue experimentation, to choose an appropriate or favourite expression system. Similarly, once the primary amino acid sequence for the trimeric polypeptide of the present invention is chosen, one of ordinary skill in the art can easily design appropriate recombinant DNA constructs which will encode the desired proteins, taking into consideration such factors as codon biases in the chosen host, the need for secretion signal sequences in the host, the introduction of proteinase cleavage sites within the signal sequence, and the like. These recombinant DNA constructs may be inserted in-frame into any of a number of expression vectors appropriate to the chosen host. The choice of an appropriate or favourite expression vector is, again, a matter well within the ability and discretion of the skilled practitioner. Preferably, the expression vector will include a strong promoter to drive expression of the recombinant constructs. Finally, the trimeric polypeptide may be isolated using suitable standard procedures well known in the art, and optionally subjected to further processing such as e.g. lyophilization.

The trimeric polypeptide according to the invention may be used for the preparation of a pharmaceutical composition by any suitable method well known in the art. The composition may together with the trimeric polypeptide, comprise one or more acceptable carriers therefore, and optionally other therapeutic ingredients. The carriers must be acceptable in the sense of being compatible with the other ingredients and not deleterious to the recipient thereof. In general, methods for the preparation of pharmaceutical compositions include the step of bringing into association the active ingredient and a carrier.

The therapeutic application of the present invention comprises the treatment of a disease or disorder in an animal, by administering a therapeutically effective amount of the trimeric polypeptide according to the invention to an animal in need thereof. As mentioned above, trimeric cytokines, and in particular trimeric cytokines of the TNF ligand super family, are known to be pleiotropic polypeptides that act as intercellular signal transduction molecules by i.a. controlling and coordinating immune and inflammatory responses. Thus, it will be appreciated that the trimeric polypeptide of the present invention may be applicable for the treatment of wide range of disorders and diseases mediated by trimeric cytokines, including inflammatory diseases, autoimmune disorders, immune disorders, infections, malignancies, and neurogenerative diseases. In one useful embodiment the disease is a pathology mediated by tumor necrosis factor, such as rheumatoid arthritis, psoriasis and Crohn's disease.

The trimeric polypeptide of the present invention may be directly administered to the animal by any suitable technique, including parenterally, and can be administered locally or systemically. The specific route of administration depends, e.g., on the medical history of the animal. Examples of parenteral administration include subcutaneous, intramuscular, intravenous, intraarterial, and intraperitoneal administration.

The animals potentially treatable by the trimeric fusion protein herein include mammals such as a human.

Finally, the present invention provides an assay method for detecting a trimeric polypeptide cytokine in a sample which comprises (i) contacting the sample with a trimeric polypeptide according to the invention, and (ii) detecting the binding of the trimeric polypeptide to the trimeric cytokine.

The invention will now be described by way of illustration in the following non-limiting examples and figures.

### Description of the figures

**Figure 1** shows the binding of a trimeric cytokine within the Tumour Necrosis Factor Super Family to a corresponding cell bound monomeric receptor, which initiates the further recruitment of two more cytokine receptors resulting in the formation of a hexameric cytokine-receptor signalling unit.
**Figure 2** shows the binding of a bivalent binder to a trimeric cytokine within the Tumour Necrosis Factor Super Family (TNFSF), which forms a 1:1 complex with the trimeric cytokine. When the bivalent binder binds to the trimeric TNFSF cytokine, only two out off three possible receptor binding sites are occupied by the bivalent binder molecule, and the third receptor binding site is left open. This implies that the open trimeric cytokine receptor binding site may associate with a cell surface trimeric cytokine receptor and thereby, due to high local concentration of the receptors on the cell surface, initiate the further recruitment of trimeric receptors and thereby signal transduction.

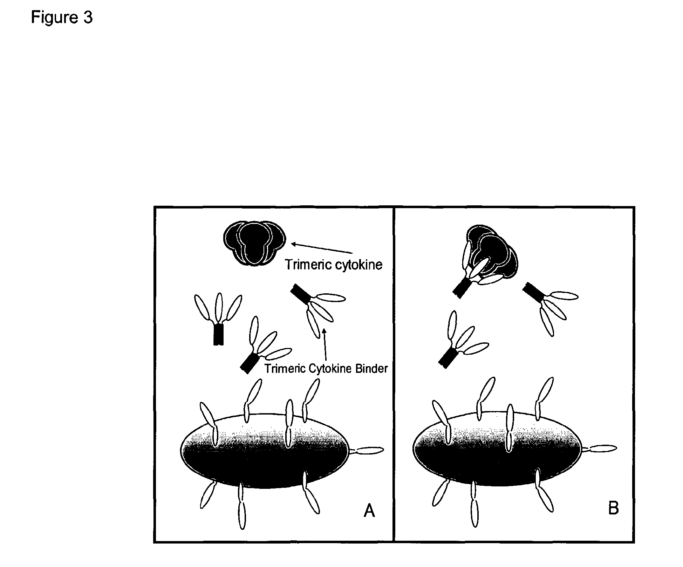

**Figure 3** shows a binder for trimeric cytokines which is capable of forming a 1:1 complex with a trimeric cytokine and at the same time effectively binding all three receptor binding sites of the trimeric cytokine, whereby no receptor binding sites are left open. The docking of the cytokine to the cell surface is blocked, and hence no signal transduction will occur.

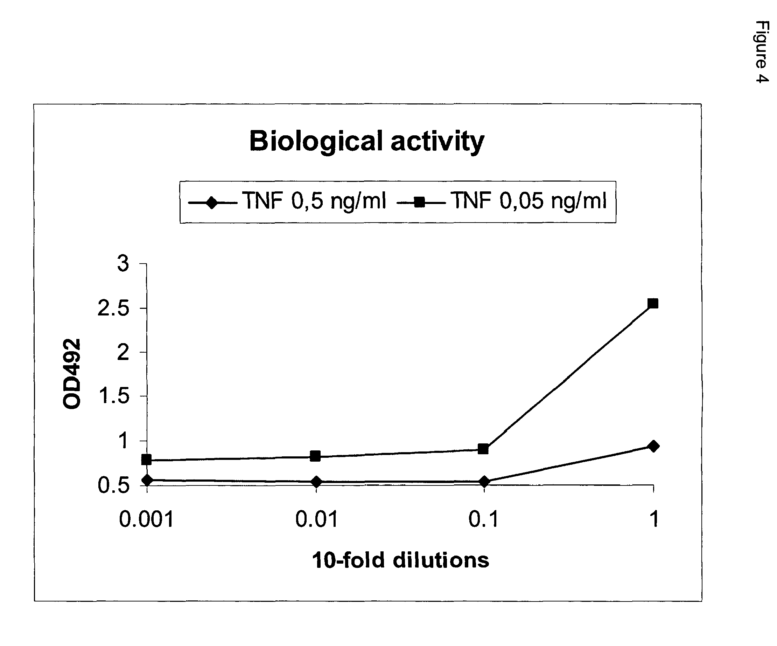

**Figure 4** shows inhibition of TNF alpha mediated cytotoxicity in a murine fibroblast cell line L929 assay at two different fixed TNF alpha concentrations (1.0 ng/ml and 0.1 ng/ml) for the trimerised receptorfragment D1 D4GSSI10 Trip in 10-fold dilutions.

### Examples

### EXAMPLE 1

### Design and construction of trip E. coli expression plasmids and phagemids for the production of trimeric cytokine binders

### Phagemids

The phagemid, psktripB, was constructed by ligation of the Sfi I and Not I restricted DNA fragment sktripB amplified from the expression plasmid pTtripb with the oligonucleotide primers C-sfikpn-TRI (SEQ ID NO:1) and N-sfikpn-TRI (SEQ ID NO:2) into a Sfi I and Not I precut vector, pCANTAB 5E supplied by Amersham Pharmacia Biotech (code no. 27-9401-01) using standard procedures. The nucleotide sequences of the sktripB inserts is given as SEQ ID NO:3.

The phagemid, pskl10tripB, was constructed by ligation of the Sfi I and Not I restricted DNA fragment skl10tripB amplified from the expression plasmid pTtripb with the oligonucleotide primers C-sfikpn-TRI (SEQ ID NO:1) and N-sfikpn-I10TRI (SEQ ID NO:4) into a Sfi I and Not I precut vector, pCANTAB 5E supplied by Amersham Pharmacia Biotech (code no. 27-9401-01) using standard procedures. The nucleotide sequence of the skl10tripB inserts is given as SEQ ID NO: 5.

### Expression plasmids

The expression plasmid pT7H6-bktripB, was constructed by ligation of the BamH I and Hind I restricted DNA fragment bktripB, amplified from the expression plasmid pTtripb with the oligonucleotide primers C-bamkpn-TRI (SEQ ID NO:6) and N-bamkpn-TRI (SEQ ID NO: 7), into the BamH I and Hind I restricted expression plasmid pT7H6 using standard procedures. The nucleotide sequence of bktripB is given as SEQ ID NO:8.

The expression plasmid pT7H6-bkl10tripB, was constructed by ligation of the BamH I and Hind I restricted DNA fragment bkl10tripB, amplified from the expression plasmid pTtripb with the oligonucleotide primers C-bamkpn-TRI (SEQ ID NO: 6) and N-bamkpn-I10TRI (SEQ ID NO:9), into the BamH I and Hind I restricted expression plasmid pT7H6 using standard procedures. The nucleotide sequence of bkItripB is given as SEQ ID NO:10.

### Example 2

### Design and construction of a trimeric TNF binders using TNFRII fragments

Fragments and subunits of the TNF receptor TNFRSF1 B (TNFRII) was used for the construction of trimeric TNF binders based on the trimerising domain TripB derived from tetranectin. The design and cloning is outlined in the following:

### Cloning of TNFRSF1B fragments into Phagemid vectors

The Phagemid vector pD1D2tripB, is constructed by ligation of the Sfi I and Kpn I restricted DNA fragment D1D2 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D2kpn-fo (SEQ ID NO: 12)) into a Sfi I and Kpn I cut vector, psktripB using standard procedures. Outlines of the resulting nucleotide sequence of D1 D2, is given as SEQ ID NO:13.

The Phagemid vector pD1D2,1/6tripB, is constructed by ligation of the Sfi I and Kpn I restricted DNA fragment D1 D1/6 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) D1/6kpn-fo (SEQ ID NO: 14)) into a Sfi I and Kpn I cut vector, psktripB using standard procedures. Outlines of the resulting nucleotide sequence of D1 D2,1/6, is given as SEQ ID NO:15.

The Phagemid vector pD1 D2,1/4tripB, is constructed by ligation of the Sfi I and Kpn I restricted DNA fragment D1 D1/4 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/4kpn-fo (SEQ ID NO: 16)) into a Sfi I and Kpn I cut vector, psktripB using standard procedures. Outlines of the resulting nucleotide sequence of D1 D2,1/4, is given as SEQ ID NO: 17.

The Phagemid vector pD1 D2,1/3tripB, is constructed by ligation of the Sfi I and Kpn I restricted DNA fragment D1 D1/3 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/3kpn-fo (SEQ ID NO: 18)) into a Sfi I and Kpn I cut vector, psktripB using standard procedures. Outlines of the resulting nucleotide sequence of D1 D2,1/3, is given as SEQ ID NO:19.

The Phagemid vector pD1 D2,1/2tripB, is constructed by ligation of the Sfi I and Kpn I restricted DNA fragment D1 D1/2 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/2kpn-fo (SEQ ID NO: 20)) into a Sfi I and Kpn I cut vector, psktripB using standard procedures. Outlines of the resulting nucleotide sequence of D1 D2,1/2, is given as SEQ ID NO:21.

The Phagemid vector pD1 D4tripB, is constructed by ligation of the Sfi I and Kpn I restricted DNA fragment D1 D4 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D4kpn-fo (SEQ ID NO: 22)) into a Sfi I and Kpn I cut vector, psktripB using standard procedures. Outlines of the resulting nucleotide sequence of D1 D4, is given as SEQ ID NO:23.

The Phagemid vector pD1D2I10tripB, is constructed by ligation of the Sfi I and Kpn I restricted DNA fragment D1 D2 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D2kpn-fo (SEQ ID NO: 12)) into a Sfi I and Kpn I cut vector, pskI10tripB using standard procedures. Outlines of the resulting nucleotide sequence of D1D2, is given as SEQ ID NO:13.

The Phagemid vector pD1 D2,1/6I10tripB, is constructed by ligation of the Sfi I and Kpn I restricted DNA fragment D1 D1/6 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/6kpn-fo (SEQ ID NO: 14)) into a Sfi I and Kpn I cut vector, pskI10tripB using standard procedures. Outlines of the resulting nucleotide sequence of D1 D2,1/6, is given as SEQ ID NO:15.

The Phagemid vector pD1D2,1/4110tripB, is constructed by ligation of the Sfi I and Kpn I restricted DNA fragment D1 D1/4 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/4kpn-fo (SEQ ID NO: 16)) into a Sfi I and Kpn I cut vector, pskI10tripB using standard procedures. Outlines of the resulting nucleotide sequence of D1D2,1/4, is given as SEQ ID NO:17.

The Phagemid vector pD1D2,1/3I10tripB, is constructed by ligation of the Sfi I and Kpn I restricted DNA fragment D1 D1/3 amplified from cDNA, isolated from a mixture of human bone marrow and human leukocyte (Clontech Laboratories, Inc cat # 7181-1 and 7182-1) (with the oligonucleotide primers D1-rev: (SEQ ID NO: 11) and D1/3kpn-fo (SEQ ID NO: 18)) into a Sfi I and Kpn I cut vector, pskI10tripB using standard procedures. Outlines of the resulting nucleotide sequence of D1D2,1/4, is given as SEQ ID NO:19.

The Phagemid vector p01D2,1/2I10tripB, is constructed by ligation of the Sfi I and Kpn I restricted DNA fragment D1D1/2 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D1/2kpn-fo (SEQ ID NO: 20)) into a Sfi I and Kpn I cut vector, pskI10tripB using standard procedures. Outlines of the resulting nucleotide sequence of D1D2,1/2, is given as SEQ ID NO:21.

The Phagemid vector pD1D4I10tripB, is constructed by ligation of the Sfi I and Kpn I restricted DNA fragment D1D4 amplified from cDNA, isolated from a mixture of human bone marrow and human leukocyte (Clontech Laboratories, Inc cat # 7181-1 and 7182-1) (with the oligonucleotide primers D1-rev (SEQ ID NO: 11) and D4kpn-fo (SEQ ID NO: 22)) into a Sfi I and Kpn I cut vector, pskI10tripB using standard procedures. Outlines of the resulting nucleotide sequence of D1D4, is given as SEQ ID NO:23.

The Phagemid vector pD2tripB, is constructed by ligation of the Sfi I and Kpn I restricted DNA fragment D2 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D2kpn-fo (SEQ ID NO: 12)) into a Sfi I and Kpn I cut vector, psktripB using standard procedures. Outlines of the resulting nucleotide sequence of D2, is given as SEQ ID NO:25.

The Phagemid vector pD2,1/6tripB, is constructed by ligation of the Sfi I and Kpn I restricted DNA fragment D1/6 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/6kpn-fo (SEQ ID NO: 14)) into a Sfi I and Kpn I cut vector, psktripB using standard procedures. Outlines of the resulting nucleotide sequence of D2,1/6, is given as SEQ ID NO:26. The Phagemid vector pD2,1/4tripB, is constructed by ligation of the Sfi I and Kpn I restricted DNA fragment D1/4 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/4kpn-fo (SEQ ID NO: 16)) into a Sfi I and Kpn I cut vector, psktripB using standard procedures. Outlines of the resulting nucleotide sequence of D2,1/4, is given as SEQ ID NO:27.

The Phagemid vector pD2,1/3tripB, is constructed by ligation of the Sfi I and Kpn I restricted DNA fragment D1/3 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/3kpn-fo (SEQ ID NO: 18)) into a Sfi I and Kpn I cut vector, psktripB using standard procedures. Outlines of the resulting nucleotide sequence of D2,1/3, is given as SEQ ID NO:28.

The Phagemid vector pD21/2tripB, is constructed by ligation of the Sfi I and Kpn I restricted DNA fragment D1/2 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/2kpn-fo (SEQ ID NO: 20)) into a Sfi I and Kpn I cut vector, psktripB using standard procedures. Outlines of the resulting nucleotide sequence of D2,1/2, is given as SEQ ID NO:29.

The Phagemid vector pD2D4tripB, is constructed by ligation of the Sfi I and Kpn I restricted DNA fragment D2D4 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D4kpn-fo (SEQ ID NO: 22)) into a Sfi I and Kpn I cut vector, psktripB using standard procedures. Outlines of the resulting nucleotide sequence of D2D4, is given as SEQ ID NO:30.

The Phagemid vector pD2I10tripB, is constructed by ligation of the Sfi I and Kpn I restricted DNA fragment D2 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D2kpn-fo (SEQ ID NO: 12)) into a Sfi I and Kpn I cut vector, pskI10tripB using standard procedures. Outlines of the resulting nucleotide sequence of D2, is given as SEQ ID NO:25.

The Phagemid vector pD2,1/6I10tripB, is constructed by ligation of the Sfi I and Kpn I restricted DNA fragment D1/6 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/6kpn-fo (SEQ ID NO: 14)) into a Sfi I and Kpn I cut vector, pskI10tripB using standard procedures. Outlines of the resulting nucleotide sequence of D2,1/6, is given as SEQ ID NO:26.

The Phagemid vector pD2,1/4I10tripB, is constructed by ligation of the Sfi I and Kpn I restricted DNA fragment D1/4 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/4kpn-fo (SEQ ID NO: 16)) into a Sfi I and Kpn I cut vector, pskI10tripB using standard procedures. Outlines of the resulting nucleotide sequence of D2,1/4, is given as SEQ ID NO:27.

The Phagemid vector pD2,1/3I10tripB, is constructed by ligation of the Sfi I and Kpn I restricted DNA fragment D1/3 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/3kpn-fo (SEQ ID NO: 18)) into a Sfi I and Kpn I cut vector, pskl10tripB using standard procedures. Outlines of the resulting nucleotide sequence of D2,1/3, is given as SEQ ID NO:28.

The Phagemid vector pD2,1/2I10tripB, is constructed by ligation of the Sfi I and Kpn I restricted DNA fragment D1/2 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D1/2kpn-fo (SEQ ID NO: 20)) into a Sfi I and Kpn I cut vector, pskI10tripB using standard procedures. Outlines of the resulting nucleotide sequence of D2,1/2, is given as SEQ ID NO:29.

The Phagemid vector pD2D4I10tripB, is constructed by ligation of the Sfi I and Kpn I restricted DNA fragment D2D4 amplified from cDNA, isolated from a mixture of human bone marrow and human leukocyte (Clontech Laboratories, Inc cat # 7181-1 and 7182-1) (with the oligonucleotide primers D2-rev (SEQ ID NO: 24) and D4kpn-fo (SEQ ID NO: 22)) into a Sfi I and Kpn I cut vector, pskI10tripB using standard procedures. Outlines of the resulting nucleotide sequence of D2D4, is given as SEQ ID NO:30.

### Cloning of TNFRSF1B (TNFRII) receptor fragments into expression vectors

The expression vector pT7H6FXD1 D2tripB, is constructed by ligation of the BamH I and Kpn I restricted DNA fragment FXD1 D2 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D2kpn-fo (SEQ ID NO: 12)) into a BamH I and Kpn I cut vector, PT7H6-bktripB using standard procedures. Outlines of the resulting nucleotide sequence of FXD1 D2, is given as SEQ ID NO:32.

The expression vector pT7H6FXD1 D2,1/6tripB, is constructed by ligation of the BamH I and Kpn I restricted DNA fragment FXD1 D1/6 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/6kpn-fo (SEQ ID NO: 14)) into a BamH I and Kpn I cut vector, PT7H6-bktripB using standard procedures. Outlines of the resulting nucleotide sequence of FXD1 D2,1/6, is given as SEQ ID NO:33.

The expression vector pT7H6FXD1 D2,1/4tripB, is constructed by ligation of the BamH I and Kpn I restricted DNA fragment FXD1 D1/4 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/4kpn-fo (SEQ ID NO: 16)) into a BamH I and Kpn I cut vector, PT7H6-bktripB using standard procedures. Outlines of the resulting nucleotide sequence of FXD1D2,1/4, is given as SEQ ID NO:34.

The expression vector pT7H6FXD1D2,1/3tripB, is constructed by ligation of the BamH I and Kpn I restricted DNA fragment FXD1D1/3 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/3kpn-fo (SEQ ID NO: 18)) into a BamH I and Kpn I cut vector, pT7H6-bktripB using standard procedures. Outlines of the resulting nucleotide sequence of FXD1D2.1/3, is given as SEQ ID NO:35.

The expression vector pT7H6FXD1D2,1/2tripB, is constructed by ligation of the BamH I and Kpn I restricted DNA fragment FXD1D1/2 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/2kpn-fo (SEQ ID NO: 20)) into a BamH I and Kpn I cut vector, pT7H6-bktripB using standard procedures. Outlines of the resulting nucleotide sequence of FXD1D2,1/2, is given as SEQ ID NO:36.

The expression vector pT7H6FXD1D4tripB, is constructed by ligation of the Sfi I and Kpn I restricted DNA fragment FXD1D4 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D4kpn-fo (SEQ ID NO: 22)) into a BamH I and Kpn I cut vector, pT7H6-bkI10tripB using standard procedures. Outlines of the resulting nucleotide sequence of FXD1D4, is given as SEQ ID NO:37.

The expression vector pT7H6FXD2tripB, is constructed by ligation of the BamH I and Kpn I restricted DNA fragment FXD2 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D2kpn-fo (SEQ ID NO: 12)) into a BamH I and Kpn I cut vector, PT7H6-bktripB using standard procedures. Outlines of the resulting nucleotide sequence of FXD2, is given as SEQ ID NO:39.

The expression vector pT7H6FXD2,1/6tripB, is constructed by ligation of the BamH I and Kpn I restricted DNA fragment FXD1/6 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) bamD2-rev (SEQ ID NO: 38) and D1/6kpn-fo (SEQ ID NO: 14)) into a BamH I and Kpn I cut vector, PT7H6-bktripB using standard procedures. Outlines of the resulting nucleotide sequence of FXD2,1/6, is given as SEQ ID NO:40.

The expression vector pT7H6FXD2,1/4tripB, is constructed by ligation of the BamH I and Kpn I restricted DNA fragment FXD1D1/4 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/4kpn-fo (SEQ ID NO: 16)) into a BamH I and Kpn I cut vector, PT7H6-bktripB using standard procedures. Outlines of the resulting nucleotide sequence of FXD2,1/4, is given as SEQ ID NO:41.

The expression vector pT7H6FXD2,1/3tripB, is constructed by ligation of the BamH I and Kpn I restricted DNA fragment FXD1D1/3 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/3kpn-fo (SEQ ID NO: 18)) into a BamH I and Kpn I cut vector, pT7H6-bktripB using standard procedures. Outlines of the resulting nucleotide sequence of FXD2,1/3, is given as SEQ ID NO:42.

The expression vector pT7H6FXD2,1/2tripB, is constructed by ligation of the BamH I and Kpn I restricted DNA fragment FXD1/2 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/2kpn-fo (SEQ ID NO: 20)) into a BamH I and Kpn I cut vector, pT7H6-bktripB using standard procedures. Outlines of the resulting nucleotide sequence of FXD2,1/2, is given as SEQ ID NO:43.

The expression vector pT7H6FXD2D4tripB, is constructed by ligation of the Sfi I and Kpn I restricted DNA fragment FXD2D4 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D4kpn-fo (SEQ ID NO: 22)) into a BamH I and Kpn I cut vector, pT7H6-bktripB using standard procedures. Outlines of the resulting nucleotide sequence of FXD2D4, is given as SEQ ID NO:44.

The expression vector pT7H6FXD1D2110tripB, is constructed by ligation of the BamH I and Kpn I restricted DNA fragment FXD1D2 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D2kpn-fo (SEQ ID NO: 12)) into a BamH I and Kpn I cut vector, PT7H6-bkI10tripB using standard procedures. Outlines of the resulting nucleotide sequence of FXD1D2, is given as SEQ ID NO:32.

The expression vector pT7H6FXD1D2,1/6110tripB, is constructed by ligation of the BamH I and Kpn I restricted DNA fragment FXD1D1/6 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/6kpn-fo (SEQ ID NO: 14)) into a BamH I and Kpn I cut vector, PT7H6-bkI10tripB using standard procedures. Outlines of the resulting nucleotide sequence of FXD1D2,1/6, is given as SEQ ID NO:33.

The expression vector pT7H6FXD1D2.1/4110tripB, is constructed by ligation of the BamH I and Kpn I restricted DNA fragment FXD1D1/4 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/4kpn-fo (SEQ ID NO: 16)) into a BamH I and Kpn I cut vector, PT7H6-bkI10tripB using standard procedures. Outlines of the resulting nucleotide sequence of FXD1D2,1/4, is given as SEQ ID NO:34.

The expression vector pT7H6FXD1D2,1/3I10tripB, is constructed by ligation of the BamH I and Kpn I restricted DNA fragment FXD1D1/3 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/3kpn-fo (SEQ ID NO: 18)) into a BamH I and Kpn I cut vector, pT7H6-bkI10tripB using standard procedures. Outlines of the resulting nucleotide sequence of FXD1D2,1/3, is given as SEQ ID NO:35.

The expression vector pT7H6FXD1D2,1/2I10tripB, is constructed by ligation of the BamH I and Kpn I restricted DNA fragment FXD1D1/2 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D1/2kpn-fo (SEQ ID NO: 20)) into a BamH I and Kpn I cut vector, pT7H6-bkI10tripB using standard procedures. Outlines of the resulting nucleotide sequence of FXD1D2, 1/2, is given as SEQ ID NO:36.

The expression vector pT7H6FXD1D4I10tripB, is constructed by ligation of the Sfi I and Kpn I restricted DNA fragment FXD1D4 amplified from cDNA, isolated from a mixture of human bone marrow and human leukocyte (Clontech Laboratories, Inc cat # 7181-1 and 7182-1) (with the oligonucleotide primers bamD1-rev (SEQ ID NO: 31) and D4kpn-fo (SEQ ID NO: 22)) into a BamH I and Kpn I cut vector, pT7H6-bkI10tripB using standard procedures. Outlines of the resulting nucleotide sequence of FXD1D4, is given as SEQ ID NO:37.

The expression vector pT7H6FXD2I10tripB, is constructed by ligation of the BamH I and Kpn I restricted DNA fragment FXD2 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D2kpn-fo (SEQ ID NO: 12)) into a BamH I and Kpn I cut vector, PT7H6-bkI10tripB using standard procedures. Outlines of the resulting nucleotide sequence of FXD2, is given as SEQ ID NO:39.

The expression vector pT7H6FXD2,1/6I10tripB, is constructed by ligation of the BamH I and Kpn I restricted DNA fragment FXD1/6 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/6kpn-fo (SEQ ID NO: 14)) into a BamH I and Kpn I cut vector, PT7H6-bkI10tripB using standard procedures. Outlines of the resulting nucleotide sequence of FXD2,1/6, is given as SEQ ID NO:40.

The expression vector pT7H6FXD2,1/4I10tripB, is constructed by ligation of the BamH I and Kpn I restricted DNA fragment FXD1D1/4 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/4kpn-fo (SEQ ID NO: 16)) into a BamH I and Kpn I cut vector, PT7H6-bkI10tripB using standard procedures. Outlines of the resulting nucleotide sequence of FXD2,1/4, is given as SEQ ID NO:41.

The expression vector pT7H6FXD2,1/3I10tripB, is constructed by ligation of the BamH I and Kpn I restricted DNA fragment FXD1D1/3 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/3kpn-fo (SEQ ID NO: 18)) into a BamH I and Kpn I cut vector, pT7H6-bkI10tripB using standard procedures. Outlines of the resulting nucleotide sequence of FXD2,1/3, is given as SEQ ID NO:42.

The expression vector pT7H6FXD2, 1/2I10tripB, is constructed by ligation of the BamH I and Kpn I restricted DNA fragment FXD1/2 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D1/2kpn-fo (SEQ ID NO: 20)) into a BamH I and Kpn I cut vector, pT7H6-bkI10tripB using standard procedures. Outlines of the resulting nucleotide sequence of FXD2,1/2, is given as SEQ ID NO:43.

The expression vector pT7H6FXD2D4I10tripB, is constructed by ligation of the Sfi I and Kpn I restricted DNA fragment FXD2D4 amplified from cDNA, isolated from a mixture of human bone marrow and human leukocyte (Clontech Laboratories, Inc cat # 7181-1 and 7182-1) (with the oligonucleotide primers bamD2-rev (SEQ ID NO: 38) and D4kpn-fo (SEQ ID NO: 22)) into a BamH I and Kpn I cut vector, pT7H6-bkI10tripB using standard procedures. Outlines of the resulting nucleotide sequence of FXD2D4, is given as SEQ ID NO:44.

The expression vector pT7AD1D4-I162-tripB, is constructed by ligation of the BamH I and Kpn I restricted DNA fragment AD1D4-I162 amplified from cDNA, isolated from a mixture of human bone marrow and human leukocyte (Clontech Laboratories, Inc cat # 7181-1 and 7182-1) (with the oligonucleotide primers pBamHI-A5 (SEQ ID NO:82) and pKpnl-I162 (SEQ ID NO:83) into a BamH I and Kpn I cut vector, pT7-bktripB using standard procedures. Outlines of the resulting nucleotide sequence AD1D4-I162-tripB, is given as SEQ ID NO:84.

The expression vector pT7AD1D4-GSS-tripB, is constructed by ligation of the BamH I and Kpn I restricted DNA fragment AD1D4-GSS amplified from cDNA, isolated from a mixture of human bone marrow and human leukocyte (Clontech Laboratories, Inc cat # 7181-1 and 7182-1) (with the oligonucleotide primers pBamHI-A5 (SEQ ID NO:82) and pKpnl-GSS (SEQ ID NO:85) into a BamH I and Kpn I cut vector, pT7-bktripB using standard procedures. Outlines of the resulting nucleotide sequence AD1D4-GSS-tripB, is given as SEQ ID NO:86.

The expression vector pT7AD1D4-D235-tripB, is constructed by ligation of the BamH I and Kpn I restricted DNA fragment AD1D4-D235 amplified from cDNA, isolated from a mixture of human bone marrow and human leukocyte (Clontech Laboratories, Inc cat # 7181-1 and 7182-1) (with the oligonucleotide primers pBamHI-A5 (SEQ ID NO:82) and pKpnl-D235 (SEQ ID NO:87) into a BamH I and Kpn I cut vector, pT7-bktripB using standard procedures. Outlines of the resulting nucleotide sequence AD1D4-D235-tripB, is given as SEQ ID NO:88.

The expression vector pT7AD1D4-I162-I10-tripB, is constructed by ligation of the BamH I and Kpn **I** restricted DNA fragment AD1D4-I162 amplified from cDNA, isolated from a mixture of human bone marrow and human leukocyte (Clontech Laboratories, Inc cat # 7181-1 and 7182-1) (with the oligonucleotide primers pBamHI-A5 (SEQ ID NO:82) and pKpnl-I162 (SEQ ID NO:83) into a BamH I and Kpn I cut vector, PT7-bkI10tripB using standard procedures. Outlines of the resulting nucleotide sequence AD1D4-I162-I10-tripB, is given as SEQ ID NO:89.

The expression vector pT7AD1D4-GSS-I10-tripB, is constructed by ligation of the BamH I and Kpn I restricted DNA fragment AD1D4-GSS amplified from cDNA, isolated from a mixture of human bone marrow and human leukocyte (Clontech Laboratories, Inc cat # 7181-1 and 7182-1) (with the oligonucleotide primers pBamHI-A5 (SEQ ID NO:83) and pKpnl-GSS (SEQ ID NO:85) into a BamH I and Kpn I cut vector, PT7-bkI10tripB using standard procedures. Outlines of the resulting nucleotide sequence AD1D4-GSS-I10-tripB is given as SEQ ID NO:90.

The expression vector pT7AD1D4-D235-I10-tripB, is constructed by ligation of the BamH I and Kpn I restricted DNA fragment AD1D4-D235 amplified from cDNA, isolated from a mixture of human bone marrow and human leukocyte (Clontech Laboratories, Inc cat # 7181-1 and 7182-1) (with the oligonucleotide primers pBamHl-A5 (SEQ ID NO:83) and pKpnl-D235 (SEQ ID NO:87) into a BamH I and Kpn I cut vector, PT7-bkI10tripB using standard procedures. Outlines of the resulting nucleotide sequence AD1D4-D235-I10-tripB, is given as SEQ ID NO:91.

The expression vector pT7AD1D4-GSS-V17-tripB, is constructed by ligation of the Kpn I and Hind III restricted DNA fragment V17-TripB amplified from cDNA (with the oligonucleotide primers pKpnl-V17 (SEQ ID NO:92) and pBAD6H (SEQ ID NO:93) into a Kpn I and Hind III cut vector, pT7AD1D4-GSS-tripB using standard procedures. Outlines of the resulting nucleotide sequence AD1D4-GSS-V17-tripB, is given as SEQ ID NO:94.

The expression vector pT7AD1D4-D235-V17-tripB, is constructed by ligation of the Kpn I and Hind III restricted DNA fragment V17-TripB amplified from cDNA (with the oligonucleotide primers pKpnl-V17 (SEQ ID NO:92) and pBAD6H (SEQ ID NO:93) into a Kpn I and Hind III cut vector, pT7AD1D4-D235-tripB using standard procedures. Outlines of the resulting nucleotide sequence AD1D4-D235-V17-tripB, is given as SEQ ID NO:95.

### Example 3

### Production, refolding and purification of trimerised TNFRII fragment AD1D4-GSS-I10-TripB

Construction of the T7 RNA polymerase dependent expression plasmid pT7D1D4GSSI10 (expressing the TNFRII derivative AD1D4-GSS-I10-TripB) is described above in Example 2.

Trimerised human TNFRII fragment AD1D4-GSS-I10 (SEQ ID NO:109) was produced by growing and expressing the plasmid pT7AD1D4-GSS-I10 in *E*. *coli* BL21 cells in a medium scale (6 x 1 litre) as described by Studier and Moffat, J. Mol. Biol., 189: 113-130, 1986. Briefly, exponentially growing cultures at 37°C were at OD600 0.8 infected with bacteriophage λ-CE6 at a multiplicity of approximately 5. Cultures were grown at 37°C for another three hours before cells were harvested by centrifugation. Cells were lysed by osmotic shock and sonification and total cellular protein extracted into phenol (adjusted to pH 8 with Trisma base). Protein was precipitated from the phenol phase by addition of 2.5 volumes of ethanol and centrifugation. The protein pellet was dissolved in a buffer containing 6 M Guanidinium chloride, 50 mM Tris-HCI pH 8 and 50 mM dithioerythriol. Following gel filtration on Sephadex G-25 (Amersham Biosciences) into 8 M Urea, 0.5 M NaCl, 50 mM Tris-HCl pH 8, and 5 mM 2-mercaptoethanol the crude protein preparation was applied to Ni²⁺ activated NTA-agarose (Qiagen, Germany) columns for purification (Hochuli et al., 1988) of the fusion proteins and, while immobilised on the column, to undergo the cyclic folding procedure as previously described in WO 94/18227.

All buffers prepared for liquid chromatography were degassed under vacuum prior to addition of reductant and/or use.

Upon application of the crude protein extracts on the Ni²⁺NTA-agarose column, the fusion protein, AD1D4-GSS-I10, was purified from the majority of coli and λ phage proteins by washing with one column volume of the loading buffer followed by 6 M Guanidinium chloride, 50 mM Tris-HCl, and 5 mM 2-mercaptoethanol until the optical density (OD) at 280 nm of the column eluate was stable.

The fusion proteins were refolded on the Ni²⁺NTA-agarose column using a gradient manager profile as described in Table 1 and 0.5 M NaCl, 50 mM Tris-HCl pH 8, and 2 mM/0.2 mM reduced/oxidized gluthatione as buffer A and 8 M urea, 0.5 M NaCl, 50 mM Tris-HCl pH 8, and 3 mM reduced gluthatione as buffer B.

After completion of the cyclic folding procedure the AD1D4-GSS-I10 fusion protein was eluted from the Ni²⁺NTA-agarose columns with a buffer containing 8 M Urea, 0.5 M NaCl, 50 mM Tris-HCl, and 10 mM EDTA pH 8.

Monomeric AD1D4-GSS-I10 fusion proteins were purified from di-mer and higher order multimers by ion exchange chromatography on S- and Q-Sepharose (AmershamBiosciences): The fusion protein eluted by the elution buffer (approximately 80 % of the fusion protein material) was gelfiltrated into a buffer containing 8 M Urea, 10 mM Tris-HCl pH 8.0 and 25 mM NaCl on Sephadex G-25 and applied onto the S-Sepharose ion exchange column. The non-binding protein in the "run through" fractions were directly loaded onto the Q-Sepharose column. Monomeric AD1D4-GSS-I10 fusion proteins were eluted from the Q-Sepharose column with a liner gradient from 8 M Urea, 25 mM NaCl, 10 mM Tris-Hcl pH 8 to 8 M Urea, 300 mM NaCl, 10 mM Tris-Hcl pH 8 over 10 column volumes. Monomeric AD1D4-GSS-I10 fusion protein eluted in the very beginning of the gradient, whereas dimers and higher order multimers eluted later. Fractions containing the monomeric fusion protein were renatured and trimerised by gelfiltration into a buffer containing 10 mM HEPES pH 7.4 and 125 mM NaCl.

**TABLE 3: Buffer Gradient manager profile**

| Step | Time (min) | Flow (ml/min) | Pump A (%) | Pump B (%) |
|---|---|---|---|---|
| 1 | 0.0 | 2.00 | 100.0 | 0.0 |
| 2 | 45.0 | 2.00 | 100.0 | 0.0 |
| 3 | 46.0 | 2.00 | 0.0 | 100.0 |
| 4 | 52.0 | 2.00 | 0.0 | 100.0 |
| 5 | 60.0 | 2.00 | 100.0 | 0.0 |
| 6 | 105.0 | 2.00 | 100.0 | 0.0 |
| 7 | 106.0 | 2.00 | 4.0 | 96.0 |
| 8 | 113.0 | 2.00 | 4.0 | 96.0 |
| 9 | 120.0 | 2.00 | 100.0 | 0.0 |
| 10 | 165.0 | 2.00 | 100.0 | 0.0 |
| 11 | 166.0 | 2.00 | 6.0 | 94.0 |
| 12 | 172.0 | 2.00 | 6.0 | 94.0 |
| 13 | 180.0 | 2.00 | 100.0 | 0.0 |
| 14 | 225.0 | 2.00 | 100.0 | 0.0 |
| 15 | 226.0 | 2.00 | 8.0 | 92.0 |
| 16 | 232.0 | 2.00 | 8.0 | 92.0 |
| 17 | 240.0 | 2.00 | 100.0 | 0.0 |
| 18 | 285.0 | 2.00 | 100.0 | 0.0 |
| 19 | 286.0 | 2.00 | 10.0 | 90.0 |
| 20 | 292.0 | 2.00 | 10.0 | 90.0 |
| 21 | 300.0 | 2.00 | 100.0 | 0.0 |
| 22 | 345.0 | 2.00 | 100.0 | 0.0 |
| 23 | 346.0 | 2.00 | 12.0 | 88.0 |
| 24 | 352.0 | 2.00 | 12.0 | 88.0 |
| 25 | 360.0 | 2.00 | 100.0 | 0.0 |
| 26 | 405.0 | 2.00 | 100.0 | 0.0 |
| 27 | 406.0 | 2.00 | 14.0 | 86.0 |
| 28 | 412.0 | 2.00 | 14.0 | 86.0 |
| 29 | 420.0 | 2.00 | 100.0 | 0.0 |
| 30 | 465.0 | 2.00 | 100.0 | 0.0 |
| 31 | 466.0 | 2.00 | 16.0 | 84.0 |
| 32 | 472.0 | 2.00 | 16.0 | 84.0 |
| 33 | 480.0 | 2.00 | 100.0 | 0.0 |
| 34 | 525.0 | 2.00 | 100.0 | 0.0 |
| 35 | 526.0 | 2.00 | 18.0 | 82.0 |
| 36 | 532.0 | 2.00 | 18.0 | 82.0 |
| 37 | 540.0 | 2.00 | 100.0 | 0.0 |
| 38 | 585.0 | 2.00 | 100.0 | 0.0 |
| 39 | 586.0 | 2.00 | 20.0 | 80.0 |
| 40 | 592.0 | 2.00 | 20.0 | 80.0 |
| 41 | 600.0 | 2.00 | 100.0 | 0.0 |
| 42 | 645.0 | 2.00 | 100.0 | 0.0 |
| 43 | 646.0 | 2.00 | 22.0 | 78.0 |
| 44 | 652.0 | 2.00 | 22.0 | 78.0 |
| 45 | 660.0 | 2.00 | 100.0 | 0.0 |
| 46 | 705.0 | 2.00 | 100.0 | 0.0 |
| 47 | 706.0 | 2.00 | 24.0 | 76.0 |
| 48 | 713.0 | 2.00 | 24.0 | 76.0 |
| 49 | 720.0 | 2.00 | 100.0 | 0.0 |
| 50 | 765.0 | 2.00 | 100.0 | 0.0 |
| 51 | 766.0 | 2.00 | 25.0 | 75.0 |
| 52 | 772.0 | 2.00 | 25.0 | 75.0 |
| 53 | 780.0 | 2.00 | 100.0 | 0.0 |
| 54 | 825.0 | 2.00 | 100.0 | 0.0 |
| 55 | 826.0 | 2.00 | 25.0 | 75.0 |
| 56 | 832.0 | 2.00 | 25.0 | 75.0 |
| 57 | 840.0 | 2.00 | 100.0 | 0.0 |
| 58 | 885.0 | 2.00 | 100.0 | 0.0 |
| 59 | 886.0 | 2.00 | 25.0 | 75.0 |
| 60 | 892.0 | 2.00 | 25.0 | 75.0 |
| 61 | 900.0 | 2.00 | 100.0 | 0.0 |
| 62 | 945.0 | 2.00 | 100.0 | 0.0 |
| 63 | 946.0 | 2.00 | 25.0 | 75.0 |
| 64 | 952.0 | 2.00 | 25.0 | 75.0 |
| 65 | 960.0 | 2.00 | 100.0 | 0.0 |
| 66 | 1005.0 | 2.00 | 100.0 | 0.0 |
| 67 | 1006.0 | 2.00 | 30.0 | 70.0 |
| 68 | 1012.0 | 2.00 | 30.0 | 70.0 |
| 69 | 1020.0 | 2.00 | 100.0 | 0.0 |
| 70 | 1065.0 | 2.00 | 100.0 | 0.0 |
| 71 | 1066.0 | 2.00 | 30.0 | 70.0 |
| 72 | 1072.0 | 2.00 | 30.0 | 70.0 |
| 73 | 1080.0 | 2.00 | 100.0 | 0.0 |
| 74 | 1125.0 | 2.00 | 100.0 | 0.0 |
| 75 | 1126.0 | 2.00 | 35.0 | 65.0 |
| 76 | 1132.0 | 2.00 | 35.0 | 65.0 |
| 77 | 1140.0 | 2.00 | 100.0 | 0.0 |
| 78 | 1185.0 | 2.00 | 100.0 | 0.0 |
| 79 | 1186.0 | 2.00 | 40.0 | 60.0 |
| 80 | 1192.0 | 2.00 | 40.0 | 60.0 |
| 81 | 1200.0 | 2.00 | 100.0 | 0.0 |
| 82 | 1245.0 | 2.00 | 100.0 | 0.0 |
| 83 | 1246.0 | 2.00 | 50.0 | 50.0 |
| 84 | 1252.0 | 2.00 | 50.0 | 50.0 |
| 85 | 1260.0 | 2.00 | 100.0 | 0.0 |
| 86 | 1305.0 | 2.00 | 100.0 | 0.0 |
| 87 | 1306.0 | 2.00 | 60.0 | 40.0 |
| 88 | 1312.0 | 2.00 | 60.0 | 40.0 |
| 89 | 1319.0 | 2.00 | 100.0 | 0.0 |
| 90 | 1364.0 | 2.00 | 100.0 | 0.0 |
| 91 | 1365.0 | 2.00 | 60.0 | 40.0 |
| 92 | 1371.0 | 2.00 | 60.0 | 40.0 |
| 93 | 1378.0 | 2.00 | 100.0 | 0.0 |
| 94 | 1423.0 | 2.00 | 100.0 | 0.0 |

### Example 4

### Biological activity of trimerised TNFRII fragment AD1D4-GSS-I10

The biological activity of trimerised TNFRII fragment AD1D4-GSS-I10 (produced as described in Example 3) was measured in a L929 cell assay for determining the inhibition of TNF alpha mediated cytotoxicity.

The murine fibroblast cell line L929 (ECACC no.: 85011425) was used to measure TNF alpha mediated cytotoxicity. Cells were maintained in RPMI 1640 liquid medium (Biowhittaker, UK) supplemented with 10% FBS, and antibiotics (100 U/ml penicillin, 100 µg/ml streptomycin). The cells were plated into 96-well flat-bottom microtiter plates (NUNC, Roskilde, Denmark) at 3 x 10⁵ cells/ml and 75 µl/well of complete RPMI 1640 medium and cultured overnight at 37°C in 5% CO₂. Recombinant human TNF alpha (rhTNFa, RD Systems, UK) (1 ng/ml and 0.1 ng/ml) was incubated with various concentrations (1 0-fold dilutions) of trimerised receptorfragment (AD1D4-GSS-I10) in complete RPMI 1640 medium supplemented with 2 µg/ml actinomycin D (Sigma) for 1 hr at 37°C in 5% CO₂. Hereafter, 75 µl/well of the TNF-alpha/trimerised receptorfragment mixtures were added to the cell culture for overnight incubation at 37°C in 5% CO₂. Each concentration of the TNF-alpha / receptorfragment mixture was tested in triplicate. Cell proliferation was determined using MTT (3-[4,5-dimethyl-thiazol-2-yl]-2,5-di-phenyl-tetrazolium bromide) as part of the CellTiter 96 NonRadioactive Cell Proliferation Assay Kit (Promega). For the preparation of reagents sufficient for one 96 well plate containing cells cultured in a 150 µl volume, 3.0 ml of MTS Solution was added to 150 µl of PMS. 30 µl/well of the combined MTS/PMS solution was added and the plates were incubated 1-4 hr at 37°C in 5% CO₂ before recording the absorbance at 492 nm using an ELISA plate reader. Controls of TNF alpha with the cells, cells alone and buffer control were also included in each assay.

The result of the experiment is summarised in Fig. 4. The cytotoxic effect of TNF alpha was significantly inhibited in the presence of the highest concentration of AD1D4-GSS-I10 thus demonstrating biological activity of the compound.

### Example 5

### Design, construction and production of a trimeric TNF binder using D2E7 antibody fragments.

Single chain antibody variable region fragments (scFv) of the humanised antibody D2E7 is selected as binders in the construction of a trimeric polypeptide capable of binding TNF. The trimeric binder may be constructed by amplifying the VH and VL domains of D2E7, based on the sequence disclosed in US 6,090,382.

### Design and construction of trimeric D2E7 scFv with a Gly,Thr linker between N-terminal scFv and C-terminal trimerising domain

The expression vector pT7H6FXD2E7tripB is constructed in two steps. The first step is the construction of pT7H6FX (D2E7VH)tripB. This vector is constructed by ligation of the Bgl II and Kpn I restricted DNA fragment FX(D7E2VH) amplified from DNA containing the sequence of D2E7 (with the oligonucleotide primers FXVH (SEQ ID NO: 45) and VHBamKpn (SEQ ID NO: 46)) into a BamH I Kpn I cut pT7H6bktripB vector. The second step is the construction of pT7H6FXD2E7tripB by ligation of the BamH I Kpn I restricted DNA fragment G4SVL amplified from DNA containing the sequence of D2E7 (with the oligonucleotide primers G4SVL (SEQ ID NO: 47) and VLkpn (SEQ ID NO: 48)) into a BamH I Kpn I cut pT7H6FX(D2E7VH)tripB. The nucleotide sequence of FXD2E7, is given as SEQ ID NO:49.

### Design and construction of trimeric D2E7 scFv with a Gly, Gly, Gly, Gly, Ser, Gly, Thr linker between N-terminal scFv and C-terminal trimerising domain

The expression vector pT7H6FXD2E7G4StripB is constructed in two steps. The first step is the construction of pT7H6FX(D2E7VH)tripB by ligation of the Bgl II and Kpn I restricted DNA fragment FX(D7E2VH) amplified from DNA contaning the sequence of D2E7 (with the oligonucleotide primers FXVH (SEQ ID NO: 45) and VHBamKpn (SEQ ID NO: 46)) into a BamH I Kpn I cut pT7H6bktripB vector. The second step is the construction of pT7H6FXD2E7tripB by ligation of the BamH I Kpn I restricted DNA fragment G4SVL amplified from DNA containing the sequence of D2E7 (with the oligonucleotide primers G4SVL (SEQ ID NO: 47) and VLG4Skpn (SEQ ID NO: 50)) into a BamH I Kpn I cut pT7H6FX(D2E7VH)tripB. The resulting nucleotide sequence of FXD2E7G4S, is given as SEQ ID NO:51.

### Design and construction of trimeric D2E7 scFv with Gly, Ser linker between the N-terminal trimerising domain and the C-terminal scFv

The Expression vector pT76HFXTripAGSD2E7, is constructed by ligation of the Bcl I and Hind III restricted DNA fragment amplified from pT7H6FXD2E7G4StripB (with the oligonucleotide primers bcIVH (SEQ ID NO:52) and VLhind (SEQ ID: 53)) into a BamH I and Hind III cut vector, pT76HFXtripa (Lorentsen, RH. et al, Biochem J.;347:83-87, 2000) using standard procedures. The resulting nucleotide sequence of GSD2E7, is given as SEQ ID NO:54.

### Design and construction of trimeric D2E7 scFv with Gly, Ser, Gly, Gly, Gly, Gly, Ser linker between the N-terminal trimerising domain and the C-terminal scFv

The Expression vector pT76HFXTripAG4SD2E7, is constructed by ligation of the Bcl I and Hind III restricted DNA fragment amplified from pT7H6FXD2E7G4StripB (with the oligonucleotide primers bclG4SVH (SEQ ID NO:55) and VLhind (SEQ ID: 53)) into a BamH I and Hind III cut vector, pT76HFXtripa (Lorentsen, RH. et al, Biochem J.;347:83-87, 2000) using standard procedures. The resulting nucleotide sequence of G4SD2E7, is given as SEQ ID NO:56.

To prepare the above fusion proteins H6FXD2E7tripB, H6FXD2E7G4StripB, H6FXTripAD2D7TripA and H6FxTripAG4SD2E7, the plasmids pT7H6FXD2E7tripB, pT7H6FXD2E7G4StripB, pT7H6FXTripAD2D7TripA and pT7H6FxTripAG4SD2E7 is grown in small scale (1 litre; 2xTY medium, 5 mM MgSO4 and 100 4 g ampicillin) in *E*. *coli* BL21 cells, as described by Studier et al. (1990). Exponentially growing cultures at 37°C are at OD600 0.8 infected with bacteriophage lambda CE6 at a multiplicity of approximately 5. Cultures are grown at 37°C for another three hours and the cells harvested by centrifugation. Cells are re-suspended in 50 ml of 0.5 M NaCl, 50 mM Tris-HCl pH 8, and 1 mM EDTA pH 8. Phenol (50 ml adjusted to pH 8) is added to each and the mixtures are sonicated to extract total protein. After clarification by centrifugation (25 minutes at 10.000 g) crude protein fractions are precipitated from the phenol phases by addition of 2.5 volumes of ethanol and centrifugation. Protein pellets are dissolved in a buffer (15-25 ml) containing 6 M guanidinium chloride, 50 mMTris-HCl pH 8 and 0.1 M dithioerythriol. Following gel filtration on Sephadex G-25 (Pharmacia, Sweden) into 8 M Urea, 1 M NaCl, 50 mM Tris-HCl pH 8 and 10 mM 2-mercaptoethanol, the crude protein preparations are applied to Ni activated NTA-agarose columns (75 ml column volume) for purification (Hochuli et al., 1988). Washing buffer (6 M guanidine-HCl, 50 mM Tris-HCl pH 8 and 10 mM 2-mercaptoethanol) is then flowed through the columns until stable baselines are obtained.

Virtually pure fusion proteins are then eluted by applying a pH gradient to each column (1000 ml gradient in 8 M urea and 10 mM 2-mercaptoethanol obtained by linear (per volume) mixing of solutions containing 50 mM sodium di-hydrogenphosphate (pH 5 buffer) and 50 mM di-sodium hydrogenphosphate (pH 8 buffer).

In preparation for in vitro refolding by the method of Thogersen et al. (WO 94/18227) 20 mg of each purified fusion protein are mixed in suspensions in refolding "buffer B" (described below) with aliquots of suspensions of Ni²⁺ activated NTA-agarose matrix sufficient to generate columns of about 75 ml packed bed volume. Each fusion protein is then subjected to the iterative refolding procedure as described for plasminogen kringle 4 in WO 94/18227, except that refolding of the scFv containing fusion proteins is carried out at 10°C using a buffer containing 0.5 M NaCl, 50 mM Tris-HCl pH 8, 2 mM glutathione and 0.2 mM oxidized glutathione as "buffer A" and a buffer containing 8 M urea, 1 M NaCl, 50 mM Tris-HCl pH 8 and 2 mM glutathione as "buffer B".

After completion of the refolding procedure each column is washed with 300 ml buffer containing 0.5 M NaCl and 50 mM Tris-HCl pH 8 to wash away glutathione. The refolded fraction of each protein is then eluted from the NTA-agarose in 20 mM EDTA, 0.5 M NaCl and 50 mM Tris-HCL pH 8 to the elution buffer. After addition of solid urea to achieve a final concentration of about 8 M to each protein sample and dilution or dialysis to reduce NaCl concentrations to below 5 mM, final purification of each correctly folded fusion protein product is then accomplished by ion exchange chromatography (S-Sepharose, Pharmacia, 1,6 (i.d.) by 90 centimeter column in a buffer containing 8 M urea, 5 mM Tris-HCl (from 1 M stock solution at pH 8) and 25 mM sodium acetate (from 1 M stock solution at pH 5), eluted at 2 ml/min). After dialysis against aqueous buffers (e.g. phosphate buffered saline) each pure and correctly refolded fusion protein is recovered in yields of 2-6 mg per litre of culture grown.

### Example 6

### Design and construction of a trimeric APRIL binder using TNFRSF13B receptor fragments

The APRIL receptor TNFRSF13B is used for the construction of trimeric binders against the trimeric cytokine APRIL, and based on the trimerising domain TripB derived from tetranectin. The design and cloning of such binders with different linkers is outlined in the following:

### Cloning of TNFRSF13B6

The expression vector pT7H6FXTNFRS13B6tripB, is constructed in two steps. The first step is to construct pT7H6FX?TNFSF13B by ligation of the Bgl II and Kpn I restricted DNA fragment FX?TNFRSF13B amplified from cDNA, isolated from a mixture of human bone marrow and human leukocyte (Clontech Laboratories, Inc cat # 7181-1 and 7182-1) (with the oligonucleotide primers bgliiFX?TNFRSF13B (SEQ ID NO: 57) and ?TNFRSF13Bkpn: (SEQ ID NO: 58)) into a BamH I and Kpn I cut vector, pT7H6-bktripB using standard procedures. The second step is to construct the final expression vector pT7H6FXTNFRSF13B6tripB by ligation of the Kpn I restricted DNA fragment ?TNFRSF13B6 amplified from cDNA, isolated from a mixture of human bone marrow and human leukocyte (Clontech Laboratories, Inc cat # 7181-1 and 7182-1) (with the oligonucleotide primers kpn?TNFSF13B (SEQ ID NO: 59) and ?TNFSF13B6kpn (SEQ ID NO: 60)) into a Kpn I cut pT7H6FX?TNFSF13B vector, using standard procedures. The resulting nucleotide sequence of FXTNFRSF13B6, is given as SEQ ID NO:61.

### Cloning of TNFRSF13B10

The expression vector pT7H6FXTNFRS13B10tripB, is constructed in two steps. First step is to construct pT7H6FX?TNFRSF13B by ligation of the Bgl II and Kpn I restricted DNA fragment FX?TNFRSF13B amplified from cDNA, isolated from a mixture of human bone marrow and human leukocyte (Clontech Laboratories, Inc cat # 7181-1 and 7182-1) (with the oligonucleotide primers bgliiFX?TNFRSF13B (SEQ ID NO: 57) and ?TNFRSF13Bkpn (SEQ ID NO: 58)) into a BamH I and Kpn I cut vector, PT7H6-bktripB using standard procedures. The second step is to construct the final expression vector pT7H6FXTNFRSF13B10tripB by ligation of the Kpn I restricted DNA fragment ?TNFSF13B10 amplified from cDNA, isolated from a mixture of human bone marrow and human leukocyte (Clontech Laboratories, Inc cat # 7181-1 and 7182-1) (with the oligonucleotide primers kpn?TNFSF13B (SEQ ID NO: 59) and ?TNFSF13B10kpn (SEQ ID NO: 62)) into a Kpn I cut pT7H6FX?TNFRSF13B vector, using standard procedures. The resulting nucleotide sequence of FXTNFRSF13B10, is given as SEQ ID NO: 63.

### Cloning of TNFRSF13B20

The expression vector pT7H6FXTNFRS13B20tripB, is constructed in two steps. First step is to construct pT7H6FX?TNFRSF13B by ligation of the Bgl II and Kpn I restricted DNA fragment FX?TNFSF13B amplified from cDNA, isolated from a mixture of human bone marrow and human leukocyte (Clontech Laboratories, Inc cat # 7181-1 and 7182-1) (with the oligonucleotide primers bgliiFX?TNFRSF13B (SEQ ID NO: 57) and ?TNFRSF13Bkpn (SEQ ID NO: 58)) into a BamH I and Kpn I cut vector, PT7H6-bktripB using standard procedures. The second step is to construct the final expression vector pT7H6FXTNFRSF13B20tripB by ligation of the Kpn I restricted DNA fragment ?TNFSF13B20 amplified from cDNA, isolated from a mixture of human bone marrow and human leukocyte (Clontech Laboratories, Inc cat # 7181-1 and 7182-1) (with the oligonucleotide primers kpn?TNFRSF13B (SEQ ID NO: 59) and ?TNFRSF13B20kpn (SEQ ID NO: 64)) into a Kpn I cut pT7H6FX?TNFRSF13B vector, using standard procedures. The resulting nucleotide sequence of FXTNFRSF13B20, is given as SEQ ID NO:65.

### Cloning of TNFRSF13B30

The expression vector pT7H6FXTNFRS13B30tripB, is constructed in two steps. First step is to construct pT7H6FX?TNFRSF13B by ligation of the Bgl II and Kpn I restricted DNA fragment FX?TNFRSF13B amplified from cDNA, isolated from a mixture of human bone marrow and human leukocyte (Clontech Laboratories, Inc cat # 7181-1 and 7182-1) (with the oligonucleotide primers bgliiFX?TNFRSF13B (SEQ ID NO: 57) and ?TNFRSF13Bkpn (SEQ ID NO: 58)) into a BamH I and Kpn I cut vector, PT7H6-bktripB using standard procedures. The second step is to construct the final expression vector pT7H6FXTNFRSF13B30tripB by ligation of the Kpn I restricted DNA fragment ?TNFRSF13B30 amplified from cDNA, isolated from a mixture of human bone marrow and human leukocyte (Clontech Laboratories, Inc cat # 7181-1 and 7182-1) (with the oligonucleotide primers kpn?TNFRSF13B (SEQ ID NO: 59) and ?TNFRSF13B30kpn (SEQ ID NO: 66)) into a Kpn I cut pT7H6FX?TNFRSF13B vector, using standard procedures. The resulting nucleotide sequence of FXTNFRSF13B30, is given as SEQ ID NO:67.

### Cloning of TNFRSF13B61

The expression vector pT7H6FXTNFRS13B61tripB, is constructed in two steps. First step is to construct pT7H6FX?TNFRSF13B by ligation of the Bgl II and Kpn I restricted DNA fragment FX?TNFRSF13B amplified from cDNA, isolated from a mixture of human bone marrow and human leukocyte (Clontech Laboratories, Inc cat # 7181-1 and 7182-1) (with the oligonucleotide primers bgliiFX?TNFRSF13B (SEQ ID NO: 57) and ?TNFRSF13Bkpn (SEQ ID NO: 58)) into a BamH I and Kpn I cut vector, PT7H6-bktripB using standard procedures. The second step is to construct the final expression vector pT7H6FXTNFRSF13B61tripB by ligation of the Kpn I restricted DNA fragment ?TNFRSF13B61 amplified from cDNA, isolated from a mixture of human bone marrow and human leukocyte (Clontech Laboratories, Inc cat # 7181-1 and 7182-1) (with the oligonucleotide primers kpn?TNFRSF13B (SEQ ID NO: 59) and ?TNFRSF13B61 kpn (SEQ ID NO: 68)) into a Kpn I cut pT7H6FX?TNFRSF13B vector, using standard procedures. The resulting nucleotide sequence of FXTNFRSF13B61, is given as SEQ ID NO:69.

### Example 7

### Design and construction of a trimeric TWEAK binder using TNFRSF Fn14 receptor fragments

The TWEAK receptor TNFRSF Fn14 is used for the construction of a trimeric binder against the trimeric cytokine TWEAK, and based on the trimerising domain TripB derived from tetranectin. The design and cloning of the binder is outlined in the following:
The expression vector pT7H6FXFn14tripB, is constructed by ligation of the BamH I and Kpn I restricted DNA fragment FXFn14 amplified from cDNA, isolated from human heart (Clontech Laboratories, Inc cat # 7121-1) (with the oligonucleotide primers bamFn-rev (SEQ ID NO: 70) and FNkpn-fo (SEQ ID NO:71) into a BamH I and Kpn I cut vector, PT7H6-bktripB using standard procedures. Outlines of the resulting the nucleotide sequence of FXFn14, is given as SEQ ID NO:72.

### Example 8

### Design and construction of a trimeric BAFF binder using TNFRSF13C receptor fragments

The BAFF receptor TNFRSF13B is used for the construction of a trimeric binder against the corresponding trimeric cytokine BAFF, and based on the trimerising domain TripB derived from tetranectin. The design and cloning of the binder is outlined in the following:

### Cloning of trimeric TNFRSF13C fragment

The expression vector pT7H6FXTNFSF13CtripB , is constructed by ligation of the BamH I and Kpn I restricted DNA fragment FXTNFRSF13C amplified from cDNA, isolated from human Lymph Node(Clontech Laboratories, Inc cat # 7164-1) (with the oligonucleotide primers bam13C-rev (SEQ ID NO: 73) and 13Ckpn-fo (SEQ ID NO:74) into a BamH I and Kpn I cut vector, PT7H6-bktripB using standard procedures. Outlines of the resulting the nucleotide sequence of FXTNFRSF13C, is given as SEQ ID NO:75.

### Example 9

### Isolation and construction of trimerised CTLD binders for TNF

The scaffold structure of Tetranectin (SEQ ID NO:96) C-type lectin-like domains (CTLD), as previously disclosed in WO/0248189, was used for the isolation and construction of a trimeric binding units for the trimeric cytokine TNF (TNF alpha). The design and cloning of the binding units is outlined in the following:

### Library construction and primary selection

Phage display was used to isolate TNF CTLD binders. The phage display libraries used were based on the monovalent CTLD format of tetranectin (SEQ ID NO:97). The combinatorial library PhtCTLD-lb003 (WO/0248189, Example 5) and a variant of this library made essential as PhtCTLD-lb003, but including two additional randomised oligonucleotides for loop 1: TN-lib3-tprev, randomised in tetranectin (SEQ ID NO:98) amino acid residue positions 116-122 and TN-lib2-tprev (SEQ ID NO: 99) and 2 additional oligonucleotides for loop 4: TN-lib3-tpfo, randomised in position 146-149 (SEQ ID NO:100) and TN-lib2-tpfo (SEQ ID NO:101) in the assembly reaction. Two rounds of selection were performed using approximately 1 x 10¹² phage as input and 10 µg/ml biotinylated-TNF in the first round of panning and 1 µg/ml in the second.

The selections were performed as follows: 20 µl streptavidin-coated paramagnetic beads (Dynal) were washed two times in 1 ml PBS (PBS, 0.2 g KCI, 0.2 g KH₂PO₄, 8 g NaCl, 1.44 g Na₂HPO₄, 2H₂O, water to 1 I, and adjusted to pH 7.4 with NaOH), and blocked in 3% non fat dried milk for one hour at room temperature. 250 µl preblocked phage in 3% non fat dried milk/PBS were mixed with 250 µl of soluble biotinylated TNF, and allowed to incubate at 37°C for 30 minutes. After washing the streptavidin beads once in PBS, 0.1 % Tween 20 and twice in PBS, these were added to the reaction to capture antigen-bound phage. The beads were extensively washed with PBS, 0.1% Tween 20 and PBS, and antigen bound phage were eluted from the beads with 200 µl 50 mM dithiothreitol for 30 min at room temperature. Eluted phages were reinfected in exponentially growing E. *coli* TG1 cells, before plating and titration on 2xTY agar plates containing 2% glucose and 0.1 mg/ml ampicillin.

Screening of single clones from selected populations by analysis of binding to TNF alpha in ELISA was done essentially as described in WO/0248189, except that antigen used for coat in ELISA was 5µg/ml TNF.

A specific TNF alpha binder, called TN3-2, was isolated from the second round of selection (Table 4).

### Secondary library construction and affinity driven selection

It was investigated whether the TNF affinity of TN3-2 could be improved by introducing new sequence variation to TN3-2. For the construction of a library of TN3-2 mutants, the TN3-2 clone was used as a template and subjected to new randomization in loop 4 using the oligonucleotides TN-lib2-tpfo (SEQ ID NO:101) and TN-lib3-tpfo (SEQ ID NO:100) (Lib. TN3-2 loop4 r). The library construction was done essentially as described in WO/0248189.

The TN3-2loop4r library was used in 3 rounds of affinity driven selection with decreasing concentrations of biotinylated TNF (100 ng/ml to 0.1 ng/ml). Selection was done as described above. Screening for positive clones was done as described above.

### Results

The sequence of the parental clone TN3-2 was different from tetranectin in loop 1 (Tetranectin amino acids nos. 116-122) and in loop 4 (Tetranectin amino acids nos. 146-149). After three rounds of affinity driven selection on the TN3-2loop4r library, positive clones were identified by ELISA analysis, and 45 out of 48 clones were shown to bind TNF alpha. Loop 1 and loop 4 sequences of a selection of the identified TNF alpha binding clones together with their corresponding SEQ ID NOs are shown in Table 4 (TN3-2-B, TN3-2-C, TN3-2-D).

**TABLE 4: Amino acid sequences in loop 1 and loop 4 of the parental clone TN3-2 and three affinity matured clones isolated from the library TN3-2loop4r**

| Clone | SEQ ID | Loop 1 sequence | Loop 4 sequence | Other mutations^{a} |
|---|---|---|---|---|
| Tetranectin | SEQ ID NO:96 | DMAAEGT | GGKT | |
| TN3-2 | SEQ ID NO:102 | KVRSRYF | PRHT | V124 to M |
| TN3-2-B | SEQ ID NO:103 | KVRSRYF | PTNN | V124 to M, D165 to G |
| TN3-2-C | SEQ ID NO:104 | KVRSRYF | PTNR | |
| TN3-2-D | SEQ ID NO:105 | KVRSRYF | PNNR | V124 to I, D165 to G |

| | | | | |
|---|---|---|---|---|
| ^{a} Mutations outside the loop 4 sequence in tetranectin. | | | | |

### Example 10

### Affinity measurements of trimerised CTLD binders for TNF

The DNA inserts encoding the monomeric CTLD TNF alpha binders isolated as described in Example 7 were subcloned into *E*. *coli* pBAD expression vector (Invitrogen Corp., USA). The recombinant monomer TNF alpha binders were expressed in *E*. *coli* and purified from periplasm according to the manufactures instructions. Purified monomers were dialysed into 10 mM Tris pH 8.0, 150 mM NaCl, 2 mM CaCl₂ before analysis on BiaCore.

In order to express the trimeric format of the TNF alpha binders, appropriate DNA inserts encoding the TNF alpha binders (from Example 7) were cleaved with the restriction endonucleases Bgl II and Mun I, and the loop 1 and 4 containing DNA fragment subcloned into Bgl II Mun I restricted *E. coli* pT7H6FX-htlec DNA (as outlined in WO 02/48189, Example 1 and Fig. 5).

The recombinant trimeric TNF alpha binders were expressed in *E*. *coli* and purification of the unfolded fusion protein derivatives were performed essentially as described in WO 94/18227, Example 9.

Briefly, refolding of the TNF alpha binders were performed by loading the fusion proteins on Ni²⁺-charged nitrilotriacetic acid (NTA) columns in 8 M urea, 50 mM Tris-HCl pH 8.0, 500 mM NaCl, 5 mM 2-mercaptoethanol. After being loaded, the columns were subjected to a cyclic refolding procedure. Refolding buffers used were: renaturation buffer A (50 mM Tris-HCl pH 8.0, 500 mM NaCl, 2 mM CaCl₂, 2 mM reduced glutathione, and 0.2 mM oxidized glutathione) and denaturing buffer B (8 M urea, 50 mM Tris-HCl pH 8.0, 500 mM NaCl, 2 mM CaCl₂, 3 mM reduced glutathione). Refolded fusion proteins were eluted from the columns by 50 mM Tris-HCl pH 8.0, 500 mM NaCl, 10 mM EDTA and the fusion proteins were cleaved with bovine blood coagulation factor Xa (Protein Engineering Technology, Aarhus, Denmark) at 1:100 (w/w) at 4°C overnight. The cleaved proteins were taken into a 8 M urea, 25 mM sodium acetate buffer (pH 5), 50 mM NaCl, 1 mM Tris-HCl buffer by gel filtration on Sephadex G25 and loaded on 10 ml SP-Sepharose columns (Amersham Biosciences) and gradient-eluted from 50 mM to 500 M NaCl over 20 column volumes. Fractions containing cleaved and monomeric protein, as judged by non-reducing SDS/PAGE analysis, were pooled and the buffer was exchanged to 10 mM Tris pH 8.0, 150 mM NaCl, 2 mM CaCl₂ by gel filtration on PD10 columns (Amersham Biosciences).

Surface plasmon resonance (SPR) binding analysis was performed on a BlAcore 3000 instrument (BiaCore, Sweden). TNF alpha capture antibody (AHC3712, Biosource International) to be immobilized was dissolved in 10 mM NaAcetate pH 5.0 and then immobilized on a CM5 BiaCore sensor chip using the Amine Coupling kit (BiaCore, Sweden).

Binding analysis was performed at a flow rate of 5 µl/min. Before loading of the protein sample, the chip was equilibrated in 10 mM Tris pH 8.0, 150 mM NaCl, 2 mM CaCl₂, 50 µM EDTA and 0.005% Surfactant P20. TNF alpha was dissolved in 10 mM Tris pH 8.0, 150 mM NaCl, 2 mM CaCl₂ at 10 µg/mL and 10 µL was injected. Aliquots (20 µl) of TNF alpha binders in monomeric or the trimeric format were injected using the KINJECT option. Five min of dissociation were allowed before the chip was regenerated with 10 mM Glycine pH 2.5. Binding of TNF alpha binders was tested at six different protein concentrations, ranging from 1 to 200 nM. Binding data were evaluated using the BlAevaluation program version 3.2 (BiaCore).

Table 5 shows the kinetic values derived from the BiaCore data. The avidity effect of trimerising the TNF alpha binders TN3-2-B, TN3-2-C, TN3-2-D is seen from the fold increase in affinity moving from the monomeric binders to the corresponding trimeric binders (TN-2-B, TN-2-C and TN-2-D).

**TABLE 5**

| Clone | SEQ ID | kₒₙ M⁻¹s⁻¹ * | k_{off} s⁻¹ | K_{D}^{obs} | Fold increase |
|---|---|---|---|---|---|
| TN-2-B (trimeric) | SEQ ID NO:106 | 3.02 x 10⁶ | 7.36 x 10⁻⁴ | ∼ 0.24 nM | 304 |
| TN3-2-B (monomeric) | SEQ ID NO:103 | 1.4 x 10' | 0.923 | 73 nM | n.a. |
| TN-2-D (trimeric) | SEQ ID NO:107 | 5.06 x 10⁵ | 2.07 x 10⁻³ | 4.1 nM | 12 |
| TN3-2-D (monomeric) | SEQ ID NO:105 | 3.64 x 10⁵ | 0.0185 | 50 nM | n.a. |
| TN-2-C (trimeric) | SEQ ID NO:108 | 8.84 x 10⁵ | 3.53 x 10⁻³ | 4 nM | 37 |
| TN3-2-C (monomeric) | SEQ ID NO:104 | 1.34 x 10⁵ | 0.022 | 150 nM | n.a. |

By trimerizing the TNF alpha binders a significant gain in affinity (K_{D}) is obtained. For example, monomeric TN3-2-B has a very poor k_{off} value. In contrast hereto, the trimeric version of the same CTLD binder (TN-2-B), has a k_{off} value which is improved by over a 1000 fold. In general it can be seen that the off-rates are improved several fold by trimerisation. Moreover, the low affine parental clone TN3-2 of Example 7 could only be measured on BiaCore in its trimeric form, as the monomer had an affinity below detection limit.

### References

Studier and Moffat (1986), Journal of Molecular Biology 189: 113-130.
Hochuli, E., Bannwarth, W., Oöbeli, H., Gentz, R. and Stüber, D. (1988), Bio/Technology 6:1321-1325.
Lorentsen, RH. et al. (2000) Biochemical Journal 347:83-87.

Numbered embodiments according to the invention:
1. A trimeric polypeptide comprising three monomers, each of said monomers comprising a specific binding member capable of binding a trimeric cytokine, and each of said monomers comprising a trimerising domain.
2. A trimeric polypeptide according to embodiment 1, wherein the trimeric cytokine is a member of the Tumor necrosis factor ligand superfamily.
3. A trimeric polypeptide according to embodiment 2, wherein the member of the Tumor necrosis factor ligand superfamily is selected from the group consisting of LTA; TNF; LTB; TNFSF4; TNFSF5; TNFSF6; TNFSF7; TNFSF8; TNFSF9; TNFSF10; TNFSF11; TNFSF12; TNFSF13; TNFSF13B; TNFSF14; TNFSF15; and TNFSF18.
4. A trimeric polypeptide according to embodiment 2, wherein the specific binding member is a polypeptide derived from a member of the Tumor necrosis factor receptor superfamily.
5. A trimeric polypeptide according to embodiment 2, wherein the member of the Tumor necrosis factor receptor superfamily is selected from the group consisting of TNFRSF1A, TNFRSF1B, LTBR, TNFRSF4, TNFRSF5, TNFRSF6, TNFRSF6B, TNFRSF7, TNFRSF8, TNFRSF9, TNFRSF10A, TNFRSF10B, TNFRSF10C, TNFRSF10D, TNFRSF11A, TNFRSF11B, TNFRSF12, TNFRSF12L, TNFRSF13B, TNFRSF13C, TNFRSF14, TNFRSF Fn14, NGFR, TNFRSF17, TNFRSF18, TNFRSF19, TNFRSF19L, TNFRSF21, TNFRSF22, and TNFRSF23.
6. A trimeric polypeptide according to embodiment 1, wherein the specific binding member is derived from TNFRSF1A (p55 TNF receptor).
7. A trimeric polypeptide according to embodiment 1, wherein the specific binding member is derived from TNFRSF1B (p75 TNF receptor).
8. A trimeric polypeptide according to embodiment 7, wherein the specific binding member is a polypeptide comprising an amino acid sequence selected from the group consisting of TNFRSF1B 1-235 (SEQ ID NO:76), TNFRSF1B 1-185 (SEQ ID NO:77), TNFRSF1B 1-163 (SEQ ID NO:78) and TNFRSF1B 1-142 (SEQ ID NO:79).
9. A trimeric polypeptide according to embodiment 7, wherein the specific binding member is a polypeptide comprising an amino acid sequence encoded by a DNA sequence selected from the group consisting of TNFRSF1B D1D2 (SEQ ID NO:13), TNFRSF1B D1D2, 1/6 (SEQ ID NO:15), TNFRSF1B D1D2 1/4 (SEQ ID NO:17), TNFRSF1B D1D2, 1/3 (SEQ ID NO:19), TNFRSF1B D1D2, 1/2 (SEQ ID NO:21), TNFRSF1B D1D4 (SEQ ID NO:23), TNFRSF1B D2 (SEQ ID NO:25), TNFRSF1B D2, 1/6 (SEQ ID NO:26), TNFRSF1B D2, 1/4 (SEQ ID NO:27), TNFRSF1B D2, 1/3 (SEQ ID NO:28), TNFRSF1B D2, 1/2 (SEQ ID NO:29) and TNFRSF1B D2D4 (SEQ ID NO:30).
10. A trimeric polypeptide according to embodiment 1, wherein the specific binding member is an antibody or antibody fragment.
11. A trimeric polypeptide according to embodiment 10, wherein the antibody or antibody fragment binds to human TNF (SEQ ID NO:80) in at least one region selected from the regions consisting of amino acid residues 1-18, 1-20, 1-26, 1-30, 12-22, 22-31, 22-40, 36-45, 49-97, 49-98 , 56-79, 58-65, 69-97, 70-87, 76-90, 96-105, 105-128, 108-128, 110-127, 115-125, 117-128, 132-157, 135-155, 136-153, 138-149, 141-153 and 146-157.
12. A trimeric polypeptide according to embodiment 11, wherein the antibody or antibody fragment is selected from MAb 1 (ECACC 89080301), MAb 21 (ECACC 90012432), MAb 25 (ECACC 89121401), MAb 32 (ECACC 89080302), MAb 37 (ECACC 89080303), MAb 42 (ECACC 89080304), MAb 47 (ECACC 89121402), MAb 53 (ECACC 90012433) and MAb 54 (ECACC 89083103) or a fragment thereof.
13. A trimeric polypeptide according to embodiment 10, wherein the antibody is D2E7 or a fragment thereof.
14. A trimeric polypeptide according to embodiment 9, wherein the antibody is CDP 870 or a fragment thereof.
15. A trimeric polypeptide according to embodiment 1, wherein the specific binding member is a protein having the scaffold structure of C-type lectin-like domains (CTLD).
16. A trimeric polypeptide according to embodiment 15, wherein the specific binding member having the scaffold structure of C-type lectin-like domains is selected from the group consisting of TN3-2-B (SEQ ID NO:103), TN3-2-C (SEQ ID NO:104) and TN3-2-D (SEQ ID NO:105).
17. A trimeric polypeptide according to embodiment 1, wherein the trimerising domain is derived from tetranectin.
18. A trimeric polypeptide according to embodiment 17, wherein the trimerising domain derived from tetranectin comprises a sequence having at least 68% amino acid sequence identity with the sequence of SEQ ID NO:81.
19. A trimeric polypeptide according to embodiment 18, wherein the amino acid sequence identity is at least 75%, such as at least 87% including at least 92%.
20. A trimeric polypeptide according to embodiment 17, wherein the trimerising domain derived from tetranectin comprises the amino acid sequence SEQ ID NO:81.
21. A trimeric polypeptide according to embodiment 17, selected from the group consisting of TN-2-B (SEQ ID NO:106), TN-2-C (SEQ ID NO:108), TN-2-D (SEQ ID NO:107), and AD1D4-GSS-I10 (SEQ ID NO:109).
22. A trimeric polypeptide according to embodiment 1, further comprising a linker between the specific binding member and the trimerising domain.
23. A pharmaceutical composition comprising the trimeric polypeptide according to any of embodiments 1-22.
24. A method of treating a subject having a pathology mediated by a trimeric cytokine such as a tumor necrosis factor, comprising administering to said subject an effective amount of the trimeric polypeptide according to any of embodiments 1-22 or the composition according to embodiment 23.
25. A method according to embodiment 24 wherein the pathology mediated by tumor necrosis factor is selected from the group consisting of rheumatoid arthritis, psoriasis and Crohn's disease.
26. A method for the preparation of a trimeric polypeptide comprising three monomers, each of said monomers comprising a specific binding member capable of binding a trimeric polypeptide cytokine, and each of said monomers comprising a trimerising domain, said method comprising the steps of (i) culturing a host transformed with a vector encoding said trimeric polypeptide under such conditions that said trimeric polypeptide is expressed; and (ii) isolating said trimeric polypeptide.
27. A method according to embodiment 26, wherein said specific binding member comprises an amino acid sequence selected from the group consisting of TNFRSF1B 1-235 (SEQ ID NO:76), TNFRSF1B 1-185 (SEQ ID NO:77), TNFRSF1B 1-163 (SEQ ID NO:78) and TNFRSF1B 1-142 (SEQ ID NO:79).
28. A method according to embodiment 26, wherein the specific binding member is a polypeptide comprising an amino acid sequence encoded by a DNA sequence selected from the group consisting of TNFRSF1B D1D2 (SEQ ID NO:13), TNFRSF1B D1D2, 1/6 (SEQ ID NO:15), TNFRSF1B D1D2 1/4 (SEQ ID NO:17), TNFRSF1B D1D2, 1/3 (SEQ ID NO:19), TNFRSF1B D1D2, 1/2 (SEQ ID NO:21), TNFRSF1B D1D4 (SEQ ID NO:23), TNFRSF1B D2 (SEQ ID NO:25), TNFRSF1B D2, 1/6 (SEQ ID NO:26), TNFRSF1B D2, 1/4 (SEQ ID NO:27), TNFRSF1B D2, 1/3 (SEQ ID NO:28), TNFRSF1B D2, 1/2 (SEQ ID NO:29) and TNFRSF1B D2D4 (SEQ ID NO:30).
29. Use of a trimeric polypeptide according to any of embodiments 1-22.
30. Use of a trimeric polypeptide according to any of embodiments 1-22 for the preparation of a pharmaceutical composition.
31. An assay method for detecting a trimeric cytokine in a sample comprising (i) contacting said sample with a trimeric polypeptide according to embodiment 1, and (ii) detecting the binding of the trimeric polypeptide to the trimeric cytokine.

## Claims

1. A trimeric polypeptide comprising three monomers, each of said monomers comprising a specific binding member capable of binding a trimeric cytokine, and each of said monomers comprising a trimerising domain.

2. The trimeric polypeptide of claim 1, wherein the trimeric cytokine comprises a TNF homology domain (THD).

3. The trimeric polypeptide of claim 1, wherein the trimeric cytokine is macrophage migration inhibitory factor (MIF).

4. The trimeric polypeptide of claim 1, wherein the trimeric polypeptide has a binding affinity (Kd) for the trimeric cytokine that is at least 1 x 10⁻⁸ M as determined by surface plasmon resonance.

5. The trimeric polypeptide of claim 1, wherein the trimeric polypeptide has a binding affinity (Kd) for the trimeric cytokine of at least 1 x 10⁻⁹ M, 1 x 10⁻¹⁰ M, 1 x 10⁻¹¹ M, 1 x 10⁻¹² M, 1 x 10⁻¹³ M, 1 x 10⁻¹⁴ M, or 1 x 10⁻¹⁵ M as determined by surface plasmon resonance.

6. The trimeric polypeptide of claim 1, wherein the K-off rate for the trimeric polypeptide is at least 1 x 10⁻³ S⁻¹, such as at least 1 x 10⁻⁴ S⁻¹, including at least 1 x 10⁻⁵ S⁻¹, or at least 1 x 10⁻⁶ S⁻¹ as determined by surface plasmon resonance.

7. The trimeric polypeptide of claim 1, for the treatment of rheumatoid arthritis, psoriasis, or Crohn's disease.

8. The trimeric polypeptide of claim 1, wherein the trimerising domain is derived from a polypeptide comprising a collectin neck region.

9. The trimeric polypeptide of claim 1, wherein the trimerising domain comprises the amino acid sequence SEQ ID NO:81 in which the cysteine residue at position 50 is substituted with serine, threonine, methionine, or any other amino acid residue.

10. The trimeric polypeptide of claim 1, wherein the trimerising domain comprises the amino acid sequence SEQ ID NO:81 having a polyhistidine sequence, cleavage site for the blood coagulating factor Xₐ, or a C-terminal KGS sequence.

11. The trimeric polypeptide of claim 1, wherein the specific binding member is linked to either the N-terminus or C-terminus of the trimerising domain.

12. The trimeric polypeptide of claim 1, wherein a specific binding member is linked to both the N-terminus and C-terminus of the trimerising domain.
